# EUROPEAN PATENT APPLICATION

(11) **EP 1 174 426 A1**
(43) Date of publication of application: **23.01.2002**
(21) Application number: 00911300.2
(22) Date of filing: 23.03.2000
(51) Int. Cl.: C07D 209/88, C07D 307/91, C07D 333/76, C07C 303/40, C07C 311/05, C07C 311/13, A61K 31/343, A61K 31/381, A61K 31/403, A61P 3/04, A61P 3/06, A61P 3/10

(54) **METHOD FOR THE PREPARATION OF TRICYCLIC AMINO ALCOHOL DERIVATIVES THROUGH AZIDES**

(30) Priority: 01.04.1999 JP 9543099
(71) Applicant: Asahi Kasei Kabushiki Kaisha, Osaka-shi, Osaka 530-8205 (JP)
(72) Inventor: MATSUBARA, Koki, Nobeoka-shi, Miyazaki 882-0872 (JP); KIDA, Hitoshi, Nobeoka-shi, Miyazaki 882-0863 (JP)
(74) Representative: Forstmeyer, Dietmar, Dr. rer. nat., Dipl.-Chem.
(86) International application number: JP0001767
(87) International publication number: WO0059885

(57) **Abstract**

The present invention is directed to processes for the preparation of a tricyclic amino-alcohol derivative useful for treating and preventing diabetes, obesity, hyperlipidemia and the like, which compound is represented by the formula (1): 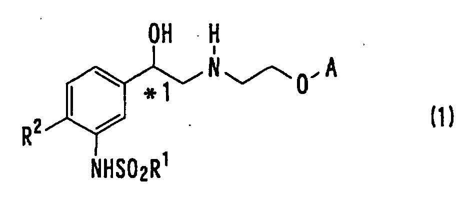 wherein R¹ represents a lower alkyl group or a benzyl group; *1 represents an asymmetric carbon atom; R² represents a hydrogen atom, a halogen atom or a hydroxyl group; and A represents one of the following groups: 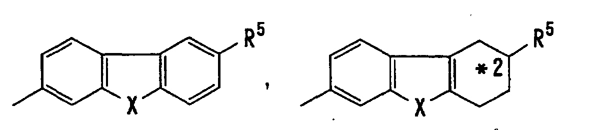 wherein X represents NH, O or S; R⁵ represents a hydrogen atom, a hydroxyl group, an amino group or an acetylamino group; and *2 represents an asymmetric carbon atom when R⁵ is not a hydrogen atom. The processes of the present invention proceed via azide derivatives and are convenient, practical preparing processes with low cost which comprise a small number of steps with good industrial work efficiency.

## Description

### FIELD OF THE INVENTION

The present invention relates to a novel process for the preparation of tricyclic amino-alcohol derivatives or salts thereof which are useful for treating and preventing diabetes, obesity, hyperlipidemia and the like and have the formula (1): 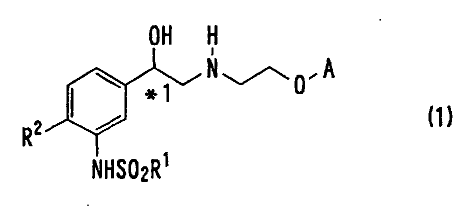 wherein
R¹ represents a lower alkyl group or a benzyl group;
*1 represents an asymmetric carbon atom;
R² represents a hydrogen atom, a halogen atom or a hydroxyl group; and
A represents one of the following groups: 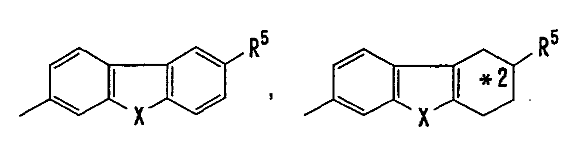
wherein X represents NH, O or S; R⁵ represents a hydrogen atom, a hydroxyl group, an amino group or an acetylamino group; and *2 represents an asymmetric carbon atom when R⁵ is not a hydrogen atom. This invention also relates to intermediates useful for the preparing process.

### BACKGROUND OF THE INVENTION

JP-A-9-249623 (WO 97/25311) and WO 99/01431 disclose in detail processes for the preparation of the compounds of the above-mentioned formula (1) and also describe that these compounds are very useful for treating and preventing diabetes, obesity, hyperlipidemia and the like.

### PROBLEMS TO BE SOLVED

However, the study on the above known processes carried out by the present inventors showed that the said processes were not necessarily a practical process. It is supposed that there is a need for a convenient, practical preparing process with low cost which comprises a small number of steps with good industrial work efficiency.

### MEANS TO SOLVE THE PROBLEMS

The study carried out by the present inventors showed some disadvantages involved in the conventional processes for preparing a compound of the formula (1) set forth above, wherein the disadvantages were that the processes required many reaction steps and several purifying works including chromatography, and did not necessarily provide a good yield. In addition, if an optical isomer, such as R-form of a compound of the formula (1) is to be finally obtained according to the synthesizing route disclosed in the above patent publications, the carbonyl group is reduced with borane as a reducing agent in the presence of a chiral auxiliary agent of the following formula (16): 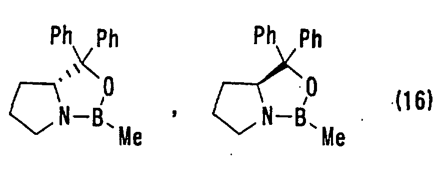 This chiral auxiliary agent is very expensive and the process for the preparation thereof is very complicated. The chiral auxiliary agent is a hazardous combustible substance and an asymmetric reduction using the said chiral auxiliary agent requires strictly anhydrous conditions, strict temperature controls, complicated works and the like, which will become problematic when the chiral auxiliary agent is industrially used.

In order to solve the above problems, the present inventors examined a variety of synthesizing processes. As a result, the present inventors have established preferred synthesizing processes successfully and completed the present invention.

That is, the present invention is a process for the preparation of a compound of the formula (1): 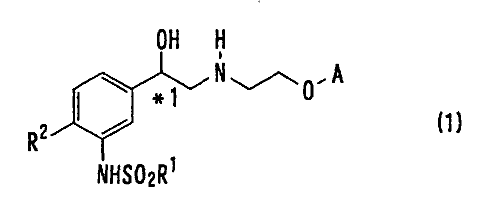 wherein R¹ represents a lower alkyl group or a benzyl group; R² represents a hydrogen atom, a halogen atom or a hydroxyl group; *1 represents an asymmetric carbon atom; and A represents one of the following groups: 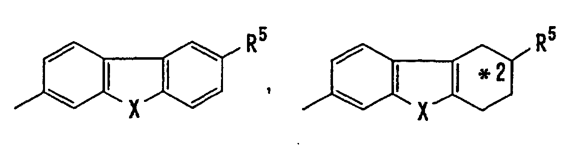 wherein X represents NH, O or S; R⁵ represents a hydrogen atom, a hydroxyl group, an amino group or an acetylamino group; and *2 represents an asymmetric carbon atom when R⁵ is not a hydrogen atom, said process comprising:
(a)
   i) azidating a compound of the formula (8): 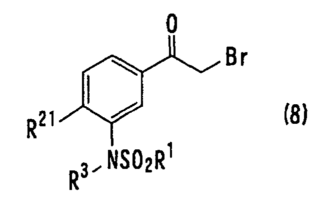 wherein R¹ is as defined above; R²¹ represents a hydrogen atom, a halogen atom, a hydroxyl group or a protected hydroxyl group; R³ represents an amino-protecting group or a hydrogen atom,
      with an azidating agent to give a compound of the formula (7): 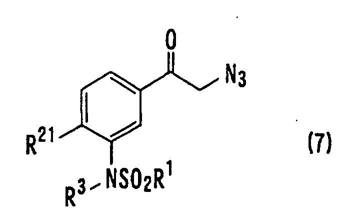 wherein R¹, R²¹ and R³ are as defined above; and then reducing the compound of the formula (7) to give a compound of the formula (5): 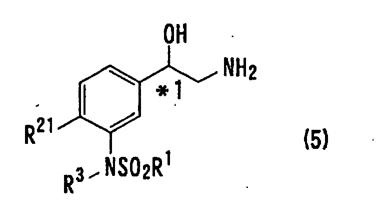 wherein R¹, R²¹, R³ and *1 are as defined above; or
   ii) asymmetrically reducing the compound of the formula (7) to give a compound of the formula (6): 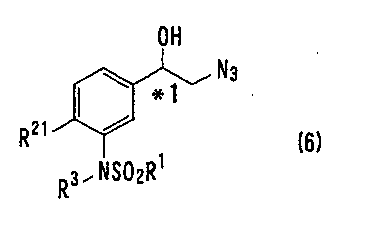 wherein R¹, R²¹, R³ and * 1 are as defined above; and then reducing the azido group to give a compound of the formula (5); or alternatively
   iii) converting the terminal bromine atom of the side-chain of a compound of the formula (8) into an amino group by amination reaction to give a compound of the formula (9): 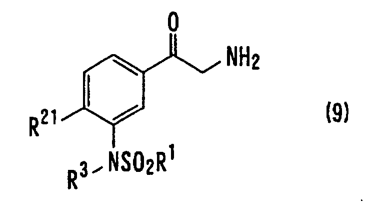 wherein R¹, R²¹ and R³ are as defined above; and then reducing the compound of the formula (9) to give a compound of the formula (5); and then,
(b) where necessary, protecting the compound of the formula (5) obtained in any one of the above steps to give a compound of the formula (4): 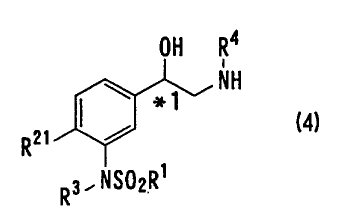 wherein R¹, R²¹, R³ and * 1 are as defined above, and R⁴ represents an amino-protecting group or a hydrogen atom; and then,
   i) reacting the compound of the formula (4) with a compound of the formula (10): 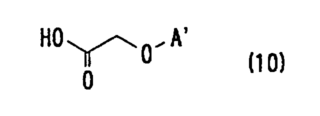 wherein A' represents one of the following groups: 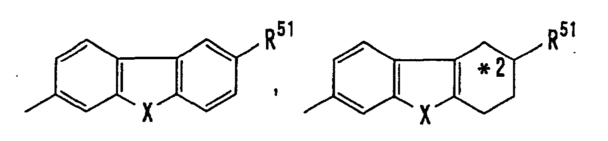 wherein X represents NH, O or S; R⁵¹ represents a hydrogen atom, a protected hydroxyl group, a protected amino group or an acetylamino group; and *2 represents an asymmetric carbon atom when R⁵¹ is not a hydrogen atom, to give a compound of the formula (3): 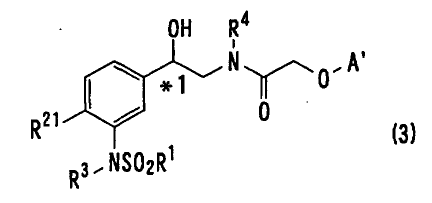 wherein R¹, R²¹, R³, R⁴, A' and * 1 are as defined above; or reacting the compound of the formula (4) with a compound of the formula (15): 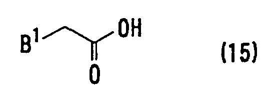 wherein B¹ represents a leaving group, to give a compound of the formula (14): 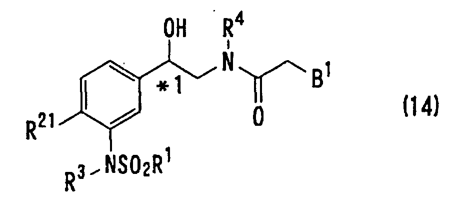 wherein R¹, R²¹, R³, R⁴, B¹ and * 1 are as defined above; and then reacting the resulting compound with a compound represented by A'-OH, wherein A' is as defined above, to give a compound of the formula (3); and then further reducing the compound of the formula (3) obtained in any one of the above steps to give a compound of the formula (2): 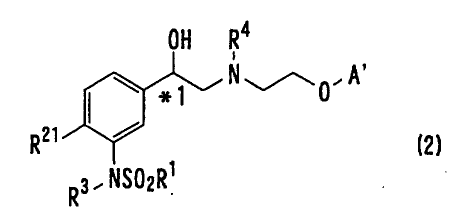 wherein R¹, R²¹, R³, R⁴, A' and *1 are as defined above; or ii) reacting the compound of the formula (4) with a compound of the formula (11): 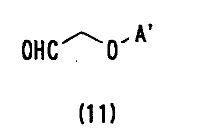 wherein A' is as defined above; and then reducing the resulting compound to give a compound of the formula (2); or
   iii) reacting the compound of the formula (4) with a compound of the formula (12): 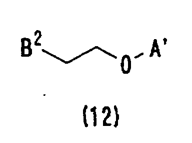
   wherein B² represents a leaving group and A' is as defined above, to give a compound of the formula (2); and then,
(c) where necessary, simultaneously or sequentially removing the protecting groups of the compound of the formula (2) obtained in any one of the above steps to give a compound of the formula (1).

The aspect of the first synthesizing route of the present invention is a process for the preparation of a compound of the formula (1): 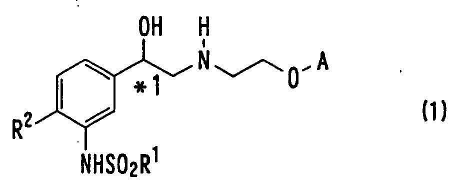 wherein R¹ represents a lower alkyl group or a benzyl group; R² represents a hydrogen atom, a halogen atom or a hydroxyl group; *1 represents an asymmetric carbon atom; and A represents one of the following groups: 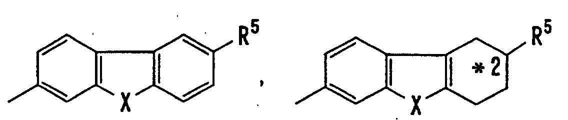 wherein X represents NH, O or S; R⁵ represents a hydrogen atom, a hydroxyl group, an amino group or an acetylamino group; and *2 represents an asymmetric carbon atom when R⁵ is not a hydrogen atom, said process comprising:
(a)
   i) azidating a compound of the formula (8): 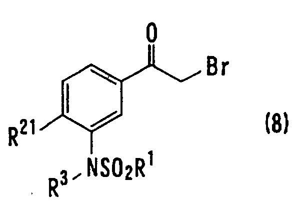 wherein R¹ is as defined above; R²¹ represents a hydrogen atom, a halogen atom, a hydroxyl group or a protected hydroxyl group; R³ represents an amino-protecting group or a hydrogen atom,
      with an azidating agent to give a compound of the formula (7): 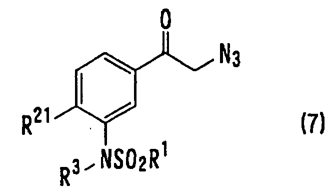 wherein R¹, R²¹ and R³ are as defined above; and then reducing the compound of the formula (7) to give a compound of the formula (5): 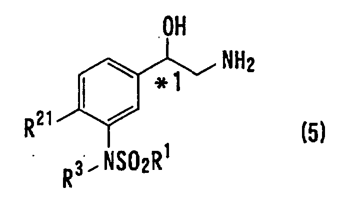 wherein R¹, R²¹, R³ and * 1 are as defined above; or
   ii) asymmetrically reducing the compound of the formula (7) to give a compound of the formula (6): 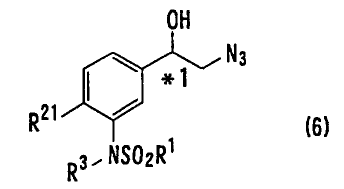 wherein R¹, R²¹, R³ and *1 are as defined above; and then reducing the azido group to give a compound of the formula (5); or alternatively
   iii) converting the terminal bromine atom of the side-chain of a compound of the formula (8) into an amino group by amination reaction to give a compound of the formula (9): 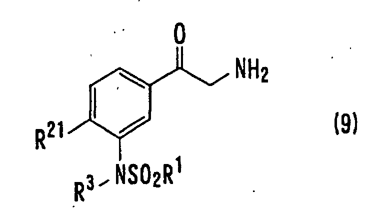 wherein R¹, R²¹ and R³ are as defined above; and then reducing the compound of the formula (9) to give a compound of the formula (5); and then,
(b) where necessary, protecting the compound of the formula (5) obtained in any one of the above steps to give a compound of the formula (4): 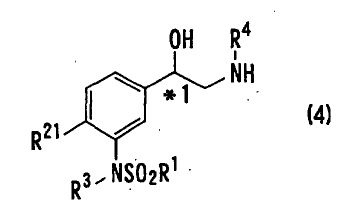 wherein R¹, R²¹, R³ and * 1 are as defined above, and R⁴ represents an amino-protecting group or a hydrogen atom; and then, reacting the compound of the formula (4) with a compound of the formula (10): 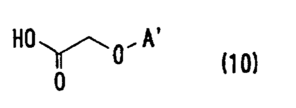 wherein A' represents one of the following groups: 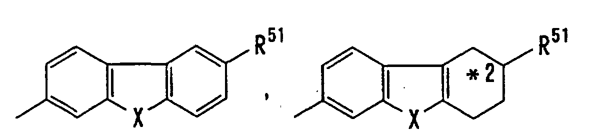 wherein X represents NH, O or S; R⁵¹ represents a hydrogen atom, a protected hydroxyl group, a protected amino group or an acetylamino group; and *2 represents an asymmetric carbon atom when R⁵¹ is not a hydrogen atom, to give a compound of the formula (3): 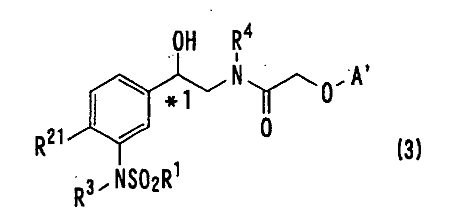 wherein R¹, R²¹, R³, R⁴, A' and * 1 are as defined above; and then further reducing the compound of the formula (3) to give a compound of the formula (2): 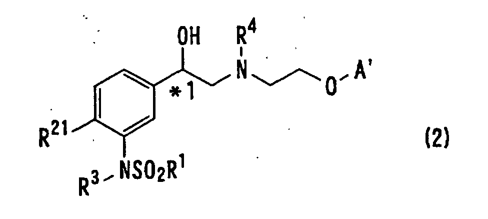 wherein R¹, R²¹, R³, R⁴, A' and *1 are as defined above; and then,
(c) where necessary, simultaneously or sequentially removing the protecting groups of the compound of the formula (2) to give a compound of the formula (1).

In the aspect of the first synthesizing route set forth above, a compound of the formula (7), a compound of the formula (6) and a compound of the formula (3) are the preferred intermediates which are novel and relatively good in crystallinity. The said compounds do not necessarily need a column chromatography purifying step and may be used in the following reaction step after being subjected to a recrystallizing treatment and the like.

Specific examples of a compound of the formula (7) include:
2-azido-1-[3-(N-benzyl-N-methylsulfonyl)amino]phenylethanone;
2-azido-1-[3-(N-benzyl-N-methylsulfonyl)amino-4-hydroxy]phenylethanone;
2-azido-1-[3-(N-benzyl-N-methylsulfonyl)amino-4-benzyloxy]phenylethanone;
2-azido-1-[3-(N-benzyl-N-methylsulfonyl)amino-4-bromo]phenylethanone;
2-azido-1-[3-(N-benzyl-N-methylsulfonyl)amino-4-chloro]phenylethanone;
2-azido-1-[3-(N-methylsulfonylamino)]phenylethanone;
2-azido-1-(4-benzyloxy-3-methylsulfonylamino)phenylethanone and the like.

Specific examples of a compound of the formula (6) include:
(±)-2-azido-1-[3-(N-benzyl-N-methylsulfonyl)amino]phenylethanol;
(±)-2-azido-1-[3-(M-benzyl-N-methylsulfonyl)amino-4-hydroxy]phenylethanol;
(±)-2-azido-1-[3-(N-benzyl-N-methylsulfonyl)amino-4-benzyloxy]phenylethanol;
(±)-2-azido-1-[4-bromo-3-(N-benzyl-N-methylsulfonyl)amino]phenylethanol;
(±)-2-azido-1-[4-chloro-3-(N-benzyl-N-methylsulfonyl)amino]phenylethanol;
(±)-2-azido-1-(3-methylsulfonylamino)phenylethanol;
(±)-2-azido-1-(4-chloro-3-methylsulfonylamino)phenylethanol;
(±)-2-azido-1-(4-benzyloxy-3-methylsulfonylamino)phenylethanol;
(R)-2-azido-1-[3-(N-benzyl-N-methylsulfonyl)amino]phenylethanol;
(R)-2-azido-1-[3-(N-benzyl-N-methylsulfonyl)amino-4-hydroxy]phenylethanol;
(R)-2-azido-1-[3-(N-benzyl-N-methylsulfonyl)amino-4-benzyloxy]phenylethanol;
(R)-2-azido-1-[4-bromo-3-(N-benzyl-N-methylsulfonyl)amino]phenylethanol;
(R)-2-azido-1-[4-chloro-3-(N-benzyl-N-methylsulfonyl)amino]phenylethanol;
(R)-2-azido-1-(3-methylsulfonylamino)phenylethanol;
(R)-2-azido-1-(4-chloro-3-methylsulfonylamino)phenylethanol;
(R)-2-azido-1-(4-benzyloxy-3-methylsulfonylamino)phenylethanol and the like.

Specific examples of a compound of the formula (3) include:
(± )-N'-[2-hydroxy-2-[3-(N-benzyl-N-methylsulfonyl)amino]phenyl]ethyl (9H-carbazol)-2-yloxyacetamide;
(±)-N'-[2-hydroxy-2-[3-(N-benzyl-N-methylsulfonyl)amino-4-benzyloxy]phenyl]ethyl (9H-carbazol)-2-yloxyacetamide;
(±)-N'-[2-hydroxy-2-[3-(N-benzyl-N-methylsulfonyl)amino-4-bromo]phenyl]ethyl (9H-carbazol)-2-yloxyacetamide;
(±)-N'-[2-hydroxy-2-[3-(N-benzyl-N-methylsulfonyl)amino-4-chloro]phenylJethyl (9H-carbazol)-2-yloxyacetamide;
(±)-N-[2-hydroxy-2-(3-methylsulfonylamino)phenyl]ethyl (9H-carbazol)-2-yloxyacetamide;
(±)-N-[2-hydroxy-2-(3-methylsulfonylamino-4-benzyloxy)phenyl]ethyl (9H-carbazol)-2-yloxyacetamide;
(±)-N-[2-hydroxy-2-(4-chloro-3-methylsulfonylamino)phenyl]ethyl (9H-carbazol)-2-yloxyacetamide;
(±)-N-[2-hydroxy-2-(3-methylsulfonylamino)phenyl]ethyl (6-benzyloxy-9H-carbazol)-2-yloxyacetamide;
(R)-N'-[2-hydroxy-2-[3-(N-benzyl-N-methylsulfonyl)amino]phenyl]ethyl (9H-carbazol)-2-yloxyacetamide;
(R)-N'-[2-hydroxy-2-[3-(N-benzyl-N-methylsulfonyl)amino-4-hydroxy]phenyl]ethyl (9H-carbazol)-2-yloxyacetamide;
(R)-N'-[2-hydroxy-2-[3-(N-benzyl-N-methylsulfonyl)amino-4-benzyloxy]phenyl]ethyl (9H-carbazol)-2-yloxyacetamide;
(R)-N'-[2-hydroxy-2-[3-(N-benzyl-N-methylsulfonyl)amino-4-bromo]phenyl]ethyl (9H-carbazol)-2-yloxyacetamide;
(R)-N'-[2-hydroxy-2-[3-(N-benzyl-N-methylsulfonyl)amino-4-chloro]phenyl]ethyl (9H-carbazol)-2-yloxyacetamide;
(R)-N-[2-hydroxy-2-(3-methylsulfonylamino)phenyl]ethyl (9H-carbazol)-2-yloxyacetamide;
(R)-N-[2-hydroxy-2-(3-methylsulfonylamino-4-chloro)phenyl]ethyl (9H-carbazol)-2-yloxyacetamide;
(R)-N-[2-hydroxy-2-(3-methylsulfonylamino-4-benzyloxy)phenyl]ethyl (9H-carbazol)-2-yloxyacetamide;
(R)-N'-[2-hydroxy-2-[3-(N-benzyl-N-methylsulfonyl)amino]phenyl]ethyl (6-benzyloxy-9H-carbazol)-2-yloxyacetamide and the like.

The aspect of the second synthesizing route of the present invention is a process for the preparation of a compound of the formula (1), said process comprising:
reacting a compound of the above-mentioned formula (4) with a compound of the formula (15) or its activated form: 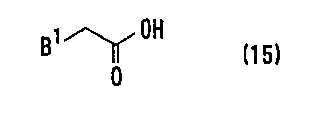 wherein B¹ represents a leaving group, to give a compound of the formula (14): 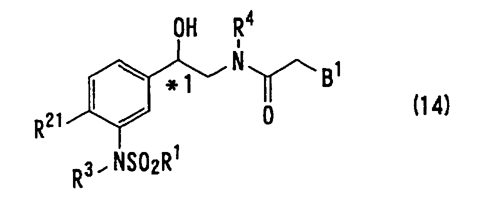 wherein R¹, R²¹, R³, R⁴, B¹ and *1 are as defined above; and
reacting the compound of the formula (14) with a compound represented by A'-OH wherein A' is as defined above, to give a compound of the formula (3); and
subjecting the thus obtained compound of the formula (3) to the sequential reaction steps as set forth above to give a compound of the formula (1) via a compound of the formula (2).

In the aspect of the second synthesizing route set forth above, a compound of the formula (14) is a preferred intermediate which is novel and relatively good in crystallinity. The said compound does not necessarily need a column chromatography purifying step and may be used in the following reaction step after being subjected to a recrystallizing treatment and the like.

Specific examples of a compound represented by the formula (14) include:
(±)-N'-[2-hydroxy-2-[3-(N-benzyl-N-methylsulfonyl)amino]phenyl]ethyl bromoacetamide;
(±)-N'-[2-hydroxy-2-[3-(N-benzyl-N-methylsulfonyl)amino-4-hydroxy]phenyl]ethyl bromoacetamide;
(±)-N'-[2-hydroxy-2-[3-(N-benzyl-N-methylsulfonyl)amino-4- benzyloxy]phenyl]ethyl bromoacetamide;
(±)-N'-[2-hydroxy-2-[3-(N-benzyl-N-methylsulfonyl)amino-4-bromo]phenyl]ethyl bromoacetamide;
(±)-N'-[2-hydroxy-2-[3-(N-benzyl-N-methylsulfonyl)amino-4-chloro]phenyl]ethyl bromoacetamide;
(±)-N-[2-hydroxy-2-(3-methylsulfonylamino)phenyl]ethyl bromoacetamide;
(±)-N-[2-hydroxy-2-(3-methylsulfonylamino-4-chloro)phenyl]ethyl bromoacetamide;
(±)-N-[2-hydroxy-2-(3-methylsulfonylamino-4-benzyloxy)phenyl]ethyl bromoacetamide;
(R)-N'-[2-hydroxy-2-[3-(N-benzyl-N-methylsulfonyl)amino]phenyl]ethyl bromoacetamide;
(R)-N'-[2-hydroxy-2-[3-(N-benzyl-N-methylsulfonyl)amino-4-hydroxy]phenyl]ethyl bromoacetamide;
(R)-N'-[2-hydroxy-2-[3-(N-benzyl-N-methylsulfonyl)amino-4-benzyloxy]phenyl]ethyl bromoacetamide;
(R)-N'-[2-hydroxy-2-[3-(N-benzyl-N-methylsulfonyl)amino-4-bromo]phenyl]ethyl bromoacetamide;
(R)-N'-[2-hydroxy-2-[3-(N-benzyl-N-methylsulfonyl)amino-4-chloro]phenyl]ethyl bromoacetamide;
(R)-N-[2-hydroxy-2-(3-methylsulfonylamino-4-chloro)phenyl]ethyl bromoacetamide;
(R)-N-[2-hydroxy-2-(3-methylsulfonylamino)phenyl]ethyl bromoacetamide;
(R)-N-[2-hydroxy-2-(3-methylsulfonylamino-4-benzyloxy)phenyl]ethyl bromoacetamide and the like.

The aspect of the third synthesizing route of the present invention is a process for the preparation of a compound of the formula (1), said process comprising:
reacting a compound of the above-mentioned formula (4) with a compound of the formula (11): 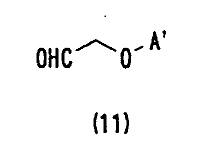 wherein A' is as defined above; and reducing the resulting compound to give a compound of the formula (2); or
reacting a compound of the above-mentioned formula (4) with a compound of the formula (12): 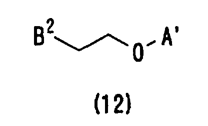 wherein B² and A' are as defined above, to give a compound of the formula (2); and
subjecting the thus obtained compound of the formula (2) to the sequential reaction steps as set forth above to give a compound of the formula (1).

### PREFERRED EMBODIMENT OF THE INVENTION

As used herein, "halogen atom" includes fluorine, chlorine, bromine and iodine, and among them, chlorine, bromine and iodine are generally preferred. Chlorine and bromine are particularly preferred.

R²¹ and R² may be a hydrogen atom, a halogen atom or a hydroxyl group (R²¹ may also be a protected hydroxyl group), with hydrogen being particularly preferred. R²¹ may be a hydroxyl group or a protected hydroxyl group, with a protected hydroxyl group being preferred. A halogen atom is also preferred as R²¹ and R². The halogen atom may be fluorine, chlorine or bromine, with chlorine and bromine being preferred.

The leaving group B¹ may be, for example, a halogen atom, a substituted sulfonyloxy group and the like. The halogen atom includes chlorine, bromine and iodine. The substituted sulfonyloxy group includes methanesulfonyloxy, p-toluenesulfonyloxy trifluoromethanesulfonyloxy and the like.

The term "lower alkyl" means a straight or branched saturated hydrocarbon containing 1 to 6 carbon atoms and may be preferably a straight or branched alkyl group, such as methyl, ethyl, n-propyl, i-propyl, n-butyl, i-butyl, sec-butyl, tert-butyl, pentyl, hexyl and the like, or a cycloalkyl group such as cyclopropyl, cyclobutyl, cyclopentyl, cyclohexyl and the like. Methyl is particularly preferred.

R¹ may be preferably the lower alkyl group set forth above, with methyl being particularly preferred. A benzyl group may be also preferred as R¹.

R³ and R⁴ may be a hydrogen atom and are preferably an amino-protecting group. Examples of the amino-protecting group include, for example, an acyl group, an acyloxy group, an easily removable aralkyl group and the like. The easily removable aralkyl group may be benzyl, substituted benzyl, naphthyl, substituted naphthyl and the like, with benzyl being particularly preferred. The aralkyl group to be used may be, for example, an aralkyl group containing 7 to 16 carbon atoms. Specific examples thereof include benzyl, phenethyl, 3-phenylpropyl, 4-phenylbutyl, (1-naphthyl)methyl, 2-(1-naphthyl)ethyl, 2-(2-naphthyl)ethyl and the like, wherein the phenyl or naphthyl moiety may have suitable substituents such as an alkyl group, an alkoxy group, a halogen atom and the like on suitable positions.

R⁵¹ and R⁵ in A' and A may be a hydrogen atom, a hydroxyl group (in case of R⁵¹, a protected hydroxyl group), an amino group (in case of R⁵¹, a protected amino group) or an acetylamino group, with hydrogen being particularly preferred. In addition, a hydroxyl group (in case of R⁵¹, a protected hydroxyl group) may be also preferred. *2 represents an asymmetric carbon atom when R⁵¹ and R⁵ is not a hydrogen atom. A compound containing *2 may be an optically active form or a racemic mixture.

A carbazole group is particularly preferred as A.

Each compound of the formulae (1), (2), (3), (4), (5), (6) and (14), in which formulae *1 means an asymmetric carbon atom, can be in the form of two optical isomers. Therefore, not only optically pure isomers of the said compounds, but also a mixture of any two isomers are encompassed in the present invention. From the viewpoint of pharmacological activity, a preferred configuration of the asymmetric carbon may be the absolute configuration R.

The hydroxyl-protecting group is not limited as long as it is commonly used as a hydroxyl-protecting group. Preferred examples of easily and selectively removable protecting group generally include, for example, a trialkylsilyl group, an alkoxyalkyl group, an acyl group and the like. These hydroxyl-protecting groups can be introduced and removed by a known method indicated in literatures accepted in the art (for example, T. W. Greene, P. G. M. Wuts, et al., Protective Groups in Organic Synthesis, Wiley-Interscience Publication). For example, a tert-butyldimethylsilyl group (TBDMS) may be introduced into the alcohol by the treatment of the alcohol with a sililating agent such as tertbutyldimethylchlorosilane, tert-butyldimethylsilyl trifluoromethanesulfonate and the like in the presence of an acid scavenger. The amount of the sililating agent to be added may be generally about 1.0 to 1.5 mol for 1 mol of the alcohol. Generally, this reaction is preferably carried out in an inert medium. The inert medium may be dichloromethane, tetrahydrofuran, acetonitrile, pyridine and the like. The inert medium may be preferably N,N-dimethylformamide. The amount of the inert medium to be used may be about 1 to 5 mL for 1 g of the alcohol. The acid scavenger may be triethylamine, N,N-diisopropylethylamine, pyridine, N,N-dimethylaminopyridine and the like. The acid scavenger may be preferably imidazole. The amount of the acid scavenger to be added may be generally about 1 to 3 mol for 1 mol of the alcohol. Generally, this reaction is preferably carried out at a temperature of about -20 to 80°C, particularly about 0°C to room temperature, for example, for 1 to 5 hours.

A benzyloxymethyl group (BOM) may be introduced into the alcohol by the treatment of the alcohol with chloromethyl benzyl ether in the presence of an acid scavenger. The amount of chloromethyl benzyl ether to be added may be generally about 1.0 to 1.5 mol for 1 mol of the alcohol. Generally, this reaction is preferably carried out in an inert medium. The inert medium may be tetrahydrofuran, acetonitrile, N,N-dimethylformamide and the like. The inert medium may be preferably dichloromethane. The amount of the inert medium to be used may be about 1 to 5 mL for 1 g of the alcohol. The acid scavenger may be triethylamine, pyridine or N,N-dimethylaminopyridine. The acid scavenger may be preferably N,N-diisopropylethylamine. The amount of the acid scavenger to be added may be generally about 1 to 3 mol for 1 mol of the alcohol. Generally, this reaction is preferably carried out at a temperature of about -20 to 80°C, particularly about 0°C to room temperature, for example, for 1 to 5 hours.

In addition, an acetyl group (Ac) may be introduced into the alcohol by the treatment of the alcohol with an acetylating agent such as acetic anhydride, acetyl chloride and the like in the presence of an acid scavenger. The amount of the acetylating agent to be added may be generally about 1 to 3 mol for 1 mol of the alcohol. Generally, this reaction is preferably carried out in an inert medium. The inert medium may be preferably tetrahydrofuran, acetonitrile, dichloromethane, pyridine and the like. The amount of the inert medium to be used may be about 1 to 5 mL for 1 g of the alcohol. The acid scavenger may be preferably triethylamine, N,N-diisopropylethylamine, pyridine, N,N-dimethylaminopyridine and the like. The amount of the acid scavenger to be added may be generally about 1 to 3 mol for 1 mol of the alcohol. Generally, this reaction is preferably carried out at a temperature of about -20 to 80°C, particularly about 0°C to room temperature, for example, for 1 to 5 hours.

The hydroxyl-protecting group may be removed as follows. For example, the tert-butyldimethylsilyl group may be removed by the treatment of the tert-butyldimethylsilyl ether with tetrabutylammonium fluoride. The amount of tetrabutylammonium fluoride to be added may be generally about 1 to 3 mol for 1 mol of the tert-butyldimethylsilyl ether. Generally, this reaction is preferably carried out in a medium such as tetrahydrofuran and the like. The amount of the medium to be used may be generally about 1 to 5 mL for 1 g of the ether. Generally, this reaction is preferably carried out at a temperature of about -20 to 60°C, particularly about 0°C to room temperature, for example, for 1 to 5 hours. This reaction is preferably carried out in the presence of acetic acid. The amount of acetic acid to be added may be generally about 1 to 2 mol for 1 mol of the tert-butyldimethylsilyl ether.

The benzyloxymethyl group may be removed by hydrogenolysis reaction using a catalyst such as palladium/carbon, palladium hydroxide/carbon and the like. The amount of the catalyst to be used may be generally about 5 to 20% by weight with respect to the benzyloxymethyl ether. Generally, this reaction is preferably carried out in a medium such as methanol, ethanol, tetrahydrofuran, acetic acid and the like. The amount of the medium to be used may be generally about 1 to 5 mL for 1 g of the benzyloxymethyl ether. Generally, this reaction is preferably carried out at a temperature of about -10 to 50°C, particularly at room temperature, for example, for 3 to 10 hours.

The acetyl group may be removed by subjecting the acetate to hydrolysis reaction using a base such as sodium carbonate, potassium carbonate, sodium hydroxide, potassium hydroxide and the like. The amount of the base to be added may be generally about 0.1 to 10 mol for 1 mol of the acetate. Generally, this reaction is preferably carried out in a medium such as methanol, ethanol, tetrahydrofuran, 1,4-dioxane, or a mixture thereof with water. The amount of the medium to be used may be generally about 1 to 5 mL for 1 g of the acetic ester. Generally, this reaction is preferably carried out at a temperature of about-20 to 100°C, particularly about 0 to 50°C, for example, for 1 to 5 hours.

In accordance with the present invention, a compound having the hydroxyl group attached to the asymmetric carbon atom *1, which is derived from the reduction of the corresponding carbonyl group, can be used in the following reactions without protection of the hydroxyl group. It is preferred, however, that such a compound is used after being protected with a suitable protecting group as occasion requires.

The amino-protecting group may be removed by a known method indicated in literatures accepted in the art (for example, T. W. Greene, P. G. M. Wuts, et al., Protective Groups in Organic Synthesis, Wiley-Interscience Publication). For example, the benzyl group may be removed by hydrogenolysis reaction using a catalyst such as palladium/carbon, palladium hydroxide/carbon and the like. The amount of the catalyst to be used may be generally about 5 to 20% by weight with respect to the protected amine. Generally, this reaction is preferably carried out in a medium such as methanol, ethanol, tetrahydrofuran, acetic acid and the like. The amount of the medium to be used may be generally about I to 5 mL for 1 g of the protected amine. Generally, this reaction is preferably carried out at a temperature of about -10 to 50°C, particularly at room temperature, for example, for 3 to 10 hours. When R²¹ is a halogen atom, however, the amino-protecting group is removed by a known method indicated in the literatures (M. Koreeda et al., Journal of Organic Chemistry, 49, p. 2081 (1984) and S. Gubert et al., Synthesis, 4, p. 318 (1991)).

The acetyl group may be removed according to the acetate hydrolyzing process using a base as set forth above. When an acyl group is used as the amino-protecting group, however, it may be preferred that this hydrolysis reaction is generally carried out at a temperature of room temperature to about 100°C.

The tert-butoxycarbonyl group (Boc) may be removed by reacting the corresponding protected amine with a known mineral acid or Lewis acid. The known mineral acid or Lewis acid may be hydrochloric acid, hydrobromic acid, sulfuric acid, acetic acid, trifluoroacetic acid, aluminium chloride, bromotrimethylsilane, iodotrimethylsilane and the like, with hydrochloric acid being preferred. The amount of the mineral acid or Lewis acid to be added can generally vary from about the same molar amount to a solvent amount with respect to the protected amine. This reaction can be carried out in a medium. However, this reaction may also be preferably carried out using the above acid as a medium. The medium may be a lower alcohol such as methanol, ethanol, n-propanol and the like, 1,4-dioxane, tetrahydrofuran, acetonitrile, dichloromethane and the like, with methanol and ethanol being preferred. Generally, this reaction is preferably carried out at a temperature of about -30 to 100°C, particularly about 0°C to room temperature, for example, for 1 to 10 hours.

The hydroxyl- and amino-protecting groups may be sequentially or simultaneously removed. For example, when R²¹ is a benzyloxy group, R³ is a benzyl group and R⁴ is a benzyl or benzyloxycarbonyl group, they can be removed under the same reaction condition and are preferably simultaneously removed. When R²¹ is a benzyloxy group and R⁴ is a tert-butoxycarbonyl group, they are removed by sequential steps comprising, for example, the first removal of the tert-butoxycarbonyl group as R⁴ followed by the removal of the benzyloxy group as R²¹. The sequence of the removals is not limited to the above and is preferably selected depending on the physical properties of the compound to be deprotected. The condition for removing each protecting group is as set forth above. In addition, these deprotections can be carried out with reference to the teachings of JP-A-9-249623.

Examples of a compound represented by the formula (1) include
2-[N-[2-(9H-carbazol-2-yloxy)]ethyl]amino-1-(3-methylsulfonylamino)phenylethanol;
2-[N-[2-(9H-carbazol-2-yloxy)]ethyl]amino-1-(4-hydroxy-3-methylsulfonylamino)phenylethanol;
2-[N-[2-(9H-carbazol-2-yloxy)]ethyl]amino-1-(4-bromo-3-methylsulfonylamino)phenylethanol;
2-[N-[2-(9H-carbazol-2-yloxy)]ethyl]amino-1-(4-chloro-3-methylsulfonylamino)phenylethanol;
2-[N-[2-(6-hydroxy-9H-carbazol-2-yloxy)]ethyl]amino-1-(3-methylsulfonylamino)phenylethanol and the like.

The most preferred examples are the above-mentioned compounds in the R-form.

The present processes for the preparation of a compound represented by the formula (1) are illustrated in more detail in the followings.

### [Preparing Process 1]

A compound of the formula (8) is azidated with an azidating agent to give a compound of the formula (7); and (i) the compound of the formula (7) is reduced to give a compound of the formula (5); or
(ii) the compound of the formula (7) is asymmetrically reduced to give a compound of the formula (6), which is further reduced to give a compound of the formula (5); or alternatively (iii) the terminal bromine atom of the side-chain of a compound of the formula (8) is converted into an amino group by amination reaction to give a compound of the formula (9), which is then reduced to give a compound of the formula (5); and then
the compound of the formula (5) obtained in any one of the above steps is, where necessarily, protected to give a compound of the formula (4); and then
the compound of the formula (4) is reacted with a compound of the formula (10) or its activated form to give a compound of the formula (3);
the compound of the formula (3) is reduced to give a compound of the formula (2); and
   where necessarily, the protecting groups are simultaneously or sequentially removed to give a compound of the formula (1).

A compound represented by the formula (8) can be synthesized, for example, by the method of A. A. Larsen et al., J. Med. Chem., 10, p. 462 (1967), or C. Kaiser et al., J. Med. Chem., 7, p. 49 (1974). Examples of a compound of the formula (8) include those of the formula (8) in which R¹ is preferably a methyl group, an ethyl group, a propyl group or a benzyl group; R²¹ is preferably a hydrogen atom, a chlorine atom, a bromine atom or a benzyloxy group; R³ is preferably a hydrogen atom or a benzyl group.

A compound of the above-mentioned formula (8) is azidated with an azidating agent. The azidating agent may be sodium azide, potassium azide and the like, and is generally used in an amount of 1 to 10 equivalents, preferably 1 to 4 equivalents, with respect to the compound of the formula (8). Generally, this reaction is preferably carried out in an inert medium. Examples of the inert medium include ether solvents such as diethyl ether, tetrahydrofuran, 1,4-dioxane and the like, ketone solvents such as acetone, methyl ethyl ketone and the like, polar solvents such as N,N-dimethylformamide, N,N-dimethylacetamide and the like, sulfoxide solvents such as dimethylsulfoxide, sulfolane and the like, nitrile solvents such as acetonitrile and the like, ester solvents such as ethyl acetate, n-butyl acetate, methyl acetate, propyl acetate and the like, alcohol solvents such as methanol, ethanol, i-propanol and the like, and halogen-containing solvents such as dichloromethane, chloroform and the like. Among them, a ketone solvent, such as acetone, methyl ethyl ketone and the like is preferably used. This reaction is generally carried out at a temperature of 0 to 100°C, preferably 10 to 30°C, for 1 to 24 hours, preferably 1 to 5 hours.

A compound of the formula (7) to be produced is characterized in that it has a good crystallinity and is easily purified. Therefore, the present process can produce a highly pure product. A compound of the formula (7) can be obtained without any purifying work such as a column chromatography purifying treatment. It may be obtained after being subjected to a simple treatment, such as a recrystallizing treatment according to a preferred example of the process of the present invention.

When a compound of the formula (7) is reduced to give a compound of the formula (6) in the form of racemic mixture, the reducing agent to be used is preferably sodium borohydride. The reducing agent is generally used in an amount of 0.5 to 5 equivalents, preferably 0.5 to 2 equivalents, with respect to the compound of the formula (7). The reaction solvent may be an alcohol solvent such as methanol, ethanol, i-propanol and the like, or a mixed solvent of the alcohol solvent with an ether solvent such as tetrahydrofuran, dioxane and the like. An alcohol solvent such as methanol, ethanol, i-propanol and the like is preferably used. The reaction temperature is generally 0 to 50°C, preferably 0 to 30°C. The reaction time is 0.5 to 10 hours, preferably 0.5 to 5 hours.

If a compound of the formula (7) is reduced with an asymmetric reduction catalyst, an optical isomer of a compound of the formula (6) can be obtained.

Further, WO 97/20789 and JP-A-9-157196 each teach a variety of processes for the preparation of an optically active alcohol from a ketone. That is, a variety of transition metals with various ligands are used as a metal complex. Most preferably, a transition metal complex is used which may be represented by MXₘLₙ wherein M represents a transition metal of the VIII group such as iron, cobalt, nickel, ruthenium, rhodium, iridium, osmium, palladium, platinum and the like; X represents a hydrogen atom, a halogen atom, a carboxyl group, a hydroxyl group, an alkoxyl group and the like; L represents a neutral ligand such as an aromatic compound, an olefin compound and the like; and, m and n represent an integer. Ruthenium is one of preferred transition metal to be contained in such transition metal complexes. When the neutral ligand is an aromatic compound, it may be a monocyclic aromatic compound. The aromatic compound may be substituted with one or more of various substituents including a hydrogen atom, a saturated or unsaturated hydrocarbon group, an allyl group, a functional group containing heteroatom(s) and the like, on any positions of the aromatic compound. The substituents may be specifically an alkyl group such as methyl, ethyl, propyl, i-propyl, butyl, tert-butyl, pentyl, hexyl, heptyl and the like; a cycloalkyl group such as cyclopropyl, cyclobutyl, cyclopentyl, cyclohexyl and the like; an unsaturated hydrocarbon group such as benzyl, vinyl, allyl and the like; or a functional group containing heteroatom(s) such as hydroxyl, alkoxyl, alkoxycarbonyl and the like.

In addition, specific examples of the metal complex include
[(R,R)-N-(p-toluenesulfonyl)-1,2-diphenylethylenediamine]benzene ruthenium complex;
[(S,S)-N-(p-toluenesulfonyl)-1,2-diphenylethylenediamine]benzene ruthenium complex;
[(R,R)-N-(p-toluenesulfonyl)-1,2-diphenylethylenediamine](p-cymene) ruthenium complex;
[(R,R)-N-(o-toluenesulfonyl)-1,2-diphenylethylenediamine](p-cymene) ruthenium complex;
[(R,R)-N-(2-mesitylenesulfonyl)-1,2-diphenylethylenediamine](p-cymene) ruthenium complex;
[(S,S)-N-(2-mesitylenesulfonyl)-1,2-diphenylethylenediamine](p-cymene) ruthenium complex;
((R,R)-N-methanesulfonyl-1,2-diphenylethylenediamine)(p-cymene) ruthenium complex;
[(R,R)-N-benzenesulfonyl-1,2-diphenylethylenediamine](p-cymene) ruthenium complex;
[(R,R)-N-(p-fluorobenzenesulfonyl)-1,2-diphenylethylenediamine](p-cymene) ruthenium complex;
[(R,R)-N-trifluoromethanesulfonyl-1,2-diphenylethylenediamine](p-cymene) ruthenium complex;
[(R,R)-N-(p-methoxybenzenesulfonyl)-1,2-diphenylethylene-diamine](p-cymene) ruthenium complex;
[(R,R)-N-methanesulfonyl-1,2-diphenylethylenediamine]mesitylene ruthenium complex;
[(R,R)-N-(p-toluenesulfonyl)-1,2-diphenylethylenediamine]mesitylene ruthenium complex;
hydride-[(R,R)-N-(p-toluenesulfonyl)-1,2-diphenylethylenediamine]benzene ruthenium complex;
hydride-[(R,R)-N-(p-toluenesulfonyl)-1,2-diphenylethylenediamine](p-cymene) ruthenium complex;
hydride-[(R,R)-N-(o-toluenesulfonyl)-1,2-diphenylethylenediamine](p-cymene) ruthenium complex;
hydride-[(R,R)-N-(2-mesitylenesulfonyl)-1,2-diphenylethylenediamine](p-cymene) ruthenium complex;
hydride-[(R,R)-N-methanesulfonyl-1,2-diphenylethylenediamine](p-cymene) ruthenium complex;
hydride-[(R,R)-N-benzenesulfonyl-1,2-diphenylethylenediamine](p-cymene) ruthenium complex;
hydride-[(R,R)-N-(p-fluorobenzenesulfonyl)-1,2-diphenylethylenediamine](p-cymene) ruthenium complex;
hydride-[(R,R)-N-trifluoromethanesulfonyl-1,2-diphenylethylenediamine](p-cymene) ruthenium complex;
hydride-[(R,R)-N-(p-methoxybenzenesulfonyl)-1,2-diphenylethylenediamine](p-cymene) ruthenium complex;
hydride-[(R,R)-N-methanesulfonyl-1,2-diphenylethylenediamine]mesitylene ruthenium complex;
hydride-[(R,R)-N-(p-toluenesulfonyl)-1,2-diphenylethylenediamine]mesitylene ruthenium complex;
chloro-[(R,R)-N-(p-toluenesulfonyl)-1,2-diphenylethylenediamine]benzene ruthenium complex;
chloro-[(R,R)-N-(p-toluenesulfonyl)-1,2-diphenylethylenediamine](p-cymene) ruthenium complex;
chloro-[(R,R)-N-(o-toluenesulfonyl)-1,2-diphenylethylenediamine](p-cymene) ruthenium complex;
chloro-[(R,R)-N-(2-mesitylenesulfonyl)-1,2-diphenylethylenediamine](p-cymene) ruthenium complex;
chloro-[(R,R)-N-methanesulfonyl-1,2-diphenylethylenediamine] (p-cymene) ruthenium complex;
chloro-[(R,R)-N-benzenesulfonyl-1,2-diphenylethylenediamine](p-cymene) ruthenium complex;
chloro-[(R,R)-N-(p-fluorobenzenesulfonyl)-1,2-diphenylethylenediamine](p-cymene) ruthenium complex;
chloro-[(R,R)-N-trifluoromethanesulfonyl-1,2-diphenylethylenediamine](p-cymene) ruthenium complex;
chloro-[(R,R)-N-(p-methoxybenzenesulfonyl)-1,2-diphenylethylenediamine](p-cymene) ruthenium complex;
chloro-[(R,R)-N-methanesulfonyl-1,2-diphenylethylenediamine]mesitylene ruthenium complex;
chloro-[(R,R)-N-(p-toluenesulfonyl)-1,2-diphenylethylenediamine]mesitylene ruthenium complex and the like.

Further, an asymmetric reduction using a catalyst obtained by the reaction of the following rhodium complex with the following chiral phosphine ligand is known. For example, [Rh(nbd)₂]ClO₄ (wherein nbd means norbornadiene), [Rh(nbd)Cl]₂, [Rh(cod)Cl]₂ (wherein cod means cycloocta-1,5-diene), and the like are known as a rhodium complex. Examples of chiral phosphine ligand include (2R,3R)-2,3-bis(diphenylphosphino)-bicyclo[2,2,1]-hept-5-ene [abbr.: (R,R)-NORPHOS]; (R)-5,5'-dimethoxy-4,4',6,6'-tetramethyl-2-diphenylphosphino-2'-dicyclohexylphosphino-1,1'-biphenyl [abbr.: (R)-MOC-BIMOP]; (R)-5,5'-dimethoxy-4,4',6,6'-tetramethyl-2,2'-bis(dicyclohexylphosphino)-1,1'-biphenyl [abbr.: (R)-Cy-BIMOP]; (2S,3S)-1,4-bis[bis(4-methoxy-3,5-dimethylphenyl)phosphino]-2,3-O-isopropylidene-2,3-butanediol [abbr.: (S,S)-MOD-DIOP]; (2S,3S)-1,4-bis(diphenylphosphino)-2,3-O-isopropylidene-2,3-butanediol [abbr.: (S,S)-DIOP]; (2S,3S)-1-diphenylphosphino-4-dicyclohexylphosphino-2,3-O-isopropylidene-2,3-butanediol [abbr.: (S,S)-DIOCP]; (R)-1-[(S)-1',2-bis(diphenylphosphino)ferrocenyl]ethanol [abbr.: (R)-(S)-BPPFOH]; (S)-1-[(S)-1',2-bis(diphenylphosphino)ferrocenyl]ethanol [abbr.: (S)-(S)-BPPFOH]; (1S,2S)-1-(diphenylphosphino)-2-[(diphenylphosphino)methyl]-cyclopentane [abbr.: (S,S)-PPCP]; (1R,2R)-1-(dicyclohexylphosphino)-2-[(diphenylphosphino)methyl]cyclopentane [abbr.: (R,R)-CPCP] and the like.

In addition, the literature (Achiwa et al. Chem. Pharm. Bull., 43, p. 748 (1995)) teaches that preferred examples of the catalyst include those obtained by reacting [Rh(cod)Cl]₂ wherein cod means cycloocta-1,5-dienea (hereinafter, optionally referred to as "rhodium complex") with a chiral phosphine ligand represented by the formula (17): 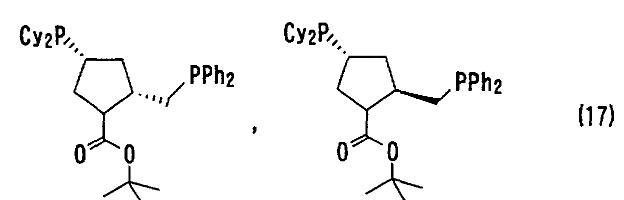 or the formula (18): 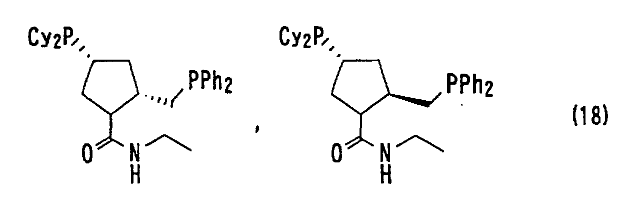 wherein Cy means a cyclohexyl group.

Further, JP-A-1-19085 teaches the preparation of rhodium-phosphine ligands, such as rhodium-1,5-cyclooctadiene-(2R,4R)-N-(tert-butoxycarbonyl)-4-dicyclohexylphosphino-2-diphenylphosphinomethylpyrrolidine complex, rhodium-1,5-cyclooctadiene-(2R,4R)-N-(methylcarbamoyl)-4-dicyclohexylphosphino-2-diphenylphosphinomethylpyrrolidine complex, rhodium-1,5-cyclooctadiene-(2R,4R)-N-(ethylcarbamoyl)-4-dicyclohexylphosphino-2-diphenylphosphinomethylpyrrolidine complex and the like. J. Org. Chem., 52, p. 5406 (1987) teaches the preparation of diisopinocampheylchloroborane.

Examples of a catalyst for asymmetric reduction catalyst which can be used for obtaining an optical isomer of a compound of the formula (6) include, for example, (diphenylethylenediamine) ruthenium complex. More specific examples thereof include [(R,R)-N-methanesulfonyl-1,2-diphenylethylenediamine](p-cymene) ruthenium complex and [(S,S)-N-methanesulfonyl-1,2-diphenylethylenediamine](p-cymene) ruthenium complex. If a compound of the formula (7) is asymmetrically reduced with (diphenylethylenediamine) ruthenium complex, the compound may be reacted with a hydrogen donating compound in the presence of the catalyst. The amount of the catalyst to be added may be generally 0.000001 to 1 equivalent, preferably 0.0001 to 0.02 equivalent, with respect to the compound of the formula (7). The hydrogen donating compound may be hydrogen gas, an alcohol solvent such as methanol, ethanol, n-propanol, i-propanol and the like, formic acid or salts thereof, an amine compound such as triethylamine and the like, an unsaturated hydrocarbon having partially saturated carbon bond such as tetralin, decalin and the like, a heterocyclic compound, hydroquinone, phosphorous acid and the like. The reaction is preferably carried out in a reaction solvent. Examples of the reaction solvent include aromatic solvents such as toluene, xylene and the like, halogen-containing solvents such as dichloromethane, chloroform and the like, polar solvents such as N,N-dimethylformamide, N,N-dimethylacetamide and the like, sulfoxide solvents such as dimethylsulfoxide, sulfolane and the like, nitrile solvents such as acetonitrile and the like, and ether solvents such as diethyl ether, tetrahydrofuran, 1,4-dioxane and the like. The reaction solvent is preferably an ether solvent such as tetrahydrofuran and the like. It is also preferred that the above mentioned hydrogen donor is used as the reaction solvent. The amount of the reaction solvent may be generally 0.1 to 100% by weight with respect to the compound of the formula (7). The reaction is carried out at a temperature of -30 to 50°C, preferably at a temperature of -20 to 10 °C with good optical yield. The reaction time is 0.5 to 10 days, preferably 1 to 3 days.

If a compound of the formula (7) is asymmetrically reduced with rhodium-phosphine ligand, the compound may be reacted with a hydrogen donating compound in the presence of the catalyst. The amount of the catalyst to be added may be generally 0.000001 to 0.1 equivalent, preferably 0.0001 to 0.1 equivalent. Hydrogen gas is used as the hydrogen donor. The reaction pressure is generally atmospheric pressure to 50 atm, preferably atmospheric pressure to 20 atm. In addition, this reaction may be carried out in the presence of a base. The base to be used may be triethylamine, diisopropylethylamine, sodium hydroxide, potassium hydroxide, lithium hydroxide, potassium methoxide, potassium tertbutoxide and the like, with an organic base such as triethylamine and the like being preferred. The amount of the base may be generally 0.0005 to 0.5 equivalent, preferably 0.005 to 0.05 equivalent, with respect to the compound of the formula (7). The reaction is preferably carried out in a reaction solvent. Examples of the reaction solvent include aromatic solvents such as toluene, xylene and the like, ether solvents such as diethyl ether, tetrahydrofuran, 1,4-dioxane and the like, alcohol solvents such as methanol, ethanol, i-propanol and the like, and water. The reaction solvent is preferably an alcohol solvent such as methanol and the like. The reaction is carried out at a temperature of 0 to 100°C, preferably 20 to 50°C. The reaction time is 0.5 to 10 days, preferably 0.5 to 2 days.

A compound of the formula (7) may be asymmetrically reduced with diisopinocampheylchloroborane. The amount of the reducing agent to be used may be generally 1.0 to 10.0 equivalents, preferably 1.0 to 3.0 equivalents with respect to the compound of the formula (7). Examples of the reaction solvent to be used include aromatic solvents such as toluene, xylene and the like, ether solvents such as diethyl ether, tetrahydrofuran, 1,4-dioxane and the like, halogen-containing solvents such as dichloromethane, chloroform and the like, and saturated aliphatic solvents such as pentane, hexane and the like. The reaction solvent is preferably an ether solvent such as tetrahydrofuran and the like. The reaction is carried out at a temperature of -50 to 50°C, preferably at a temperature of -20 to 0°C with good optical yield. The reaction time is 1.0 to 24 hours, preferably 5 to 15 hours.

If a compound of the formula (6) is reduced to prepare a compound of the formula (5), the reducing agent to be used may be lithium aluminium hydride or a hydrogen donor with a catalyst such as platinum oxide or palladium/carbon. Hydrogen gas with platinum oxide as the catalyst is preferably used in view of safety and easiness of work-up. Platinum oxide is generally used in an amount of 0.001 to 0.1 equivalent, preferably 0.01 to 0.1 equivalent with respect to the compound of the formula (6). Hydrogen gas is used as the hydrogen donor and the reaction pressure is generally atmospheric pressure to 10 atm, preferably atmospheric pressure to 5 atm. Examples of the reaction solvent include alcohol solvents such as methanol, ethanol, i-propanol and the like, and ether solvents such as diethyl ether, tetrahydrofuran, 1,4-dioxane and the like. Preferred reaction solvent is an alcohol solvent such as methanol and the like. The reaction is carried out at a temperature of 0 to 100°C, preferably 10 to 40°C. The reaction time is 1 to 50 hours, preferably 10 to 30 hours.

Further, a compound of the formula (5) may be obtained by reducing a compound of the formula (7). The reducing agent to be used may be selected from, for example, lithium aluminium hydride and hydrogen gas with a catalyst such as palladium/carbon depending on the nature of R²¹ and the like. Generally, the preferred reducing agent may be lithium aluminium hydride. The reducing agent (lithium aluminium hydride) may be generally used in an amount of 2 to 5 equivalents, preferably 2 to 3 equivalents with respect to the compound of the formula (7). The reaction solvent to be used may be ether solvents such as diethyl ether, tetrahydrofuran, 1,4-dioxane and the like. The reaction temperature may be generally 0 to 100°C, preferably 10 to 40°C. The reaction time may be generally 1 to 50 hours, preferably 10 to 30 hours. When a compound of the formula (7) in which R²¹ is a halogen atom is reduced to obtain a compound of the formula (5), borane is preferably used as the reducing agent. In this case, the amount of the reducing agent (borane) to be used may be generally 1 to 10 equivalents, preferably 2 to 4 equivalents with respect to the compound of the formula (7). Examples of the reaction solvent include ether solvents such as diethyl ether, tetrahydrofuran, 1,4-dioxane and the like, and aromatic solvents such as toluene, xylene and the like. Preferred reaction solvent may be an ether solvent such as tetrahydrofuran. The reaction temperature may be generally 0 to 100°C, preferably 10 to 40°C. The reaction time may be generally 1 to 50 hours, preferably.10 to 30 hours.

Further, a compound of the formula (5) may be directly obtained from a compound of the formula (9) by reducing the said compound. A compound of the formula (9) can be synthesized according to a known method indicated in a literature (for example, A. A. Larsen et al., J. Med. Chem., 10, p. 462 (1967) or C. Kaiser, J. Med. Chem., 7, p. 49 (1974)).

Further, a compound of the formula (9) may also be obtained by converting the terminal bromine atom of the side-chain of a compound of the formula (8) into an amino group by amination reaction. Hexamethylenetetramine, ammonia and the like may be used in a reaction for aminating a compound of the formula (8). Hexamethylenetetramine may be preferably used. If hexamethylenetetramine is used, a compound of the formula (9) may be obtained by reacting a compound of the formula (8) with hexamethylenetetramine followed by treating with an acid to form an amino group. The amount of hexamethylenetetramine, ammonia and the like to be used may be generally I to 10 equivalents, preferably 1 to 3 equivalents with respect to the compound of the formula (8). Examples of the reaction solvent to be used include halogen-containing solvents such as chloroform, methylene chloride and the like, and ether solvents such as tetrahydrofuran, 1,4-dioxane and the like. The preferred reaction solvent may be an ether solvent such as tetrahydrofuran and the like. The reaction may be carried out at a temperature of 0 to 100°C, preferably 10 to 40°C. The reaction time may be generally 1 to 50 hours, preferably 10 to 30 hours. The acid to be used after the reaction with hexamethylenetetramine may be, for example, hydrochloric acid, sulfuric acid, acetic acid and the like. The amount of the acid to be used may be generally 1 to 10 equivalents, preferably 5 to 8 equivalents with respect to the compound of the formula (8). The reaction solvents may be an alcohol solvent such as methanol, ethanol, propanol and the like. The reaction temperature may be generally 0 to 100°C, preferably 70 to 80°C. The reaction time may be generally 1 to 10 minutes, preferably 5 to 8 minutes.

The reducing reaction of a compound of the formula (9) to a compound of the formula (5) can be carried out according to the reducing reaction of a compound of the formula (7) to a compound of the formula (5) in the racemic form; the reducing reaction of a compound of the formula (7) to a compound of the formula (6) in the racemic form; the reducing reaction of a compound of the formula (6) to a compound of the formula (5); or the asymmetrically reducing reaction of a compound of the formula (7) to one of the optical isomers of a compound of the formula (6), as set forth above.

The thus obtained compound of the formula (5) by one of the above methods is preferred in that it does not necessarily need a purifying treatment such as column chromatography and may be subjected to an easy treatment such as a recrystallizing treatment.

A compound of the formula (5) is, where necessarily, subjected to a protecting treatment to give a compound of the formula (4). Protecting a compound of the formula (5) is not always necessary. A benzyl group as R⁴ can be introduced by reacting a compound of the formula (5) with benzaldehyde to give a Shiff base and then reducing the Shiff base. The amount of benzaldehyde to be used may be generally 1.0 to 5.0 equivalents, preferably 1.0 to 2.0 equivalents with respect to the compound of the formula (5). The reducing agent may be platinum oxide with a hydrogen donor, sodium borohydride, sodium cyanoborohydride and the like, with platinum oxide with a hydrogen donor being preferred. The amount of the reducing agent (platinum oxide) to be used may be generally 0.001 to 0.1 equivalent, preferably 0.01 to 0.1 equivalent. Hydrogen gas is used as the hydrogen donor generally at a reaction pressure of atmospheric pressure to 10 atm, preferably atmospheric pressure to 5 atm. Examples of the reaction solvent include alcohol solvents such as methanol, ethanol, i-propanol and the like, and ether solvents such as diethyl ether, tetrahydrofuran, 1,4-dioxane and the like. The preferred reaction solvent is an alcohol solvent such as methanol and the like. The reaction is carried out at a temperature of 0 to 100°C, preferably 10 to 40°C. The reaction time is 1 to 50 hours, preferably 10 to 30 hours.

A compound of the formula (3) may be obtained by reacting a compound of the formula (4) with a compound of the formula (10) or its activated form.

A compound of the formula (10) may be obtained by reacting a compound represented by A'-OH with a bromoacetate such as methyl bromoacetate, ethyl bromoacetate, tert-butyl bromoacetate or n-butyl bromoacetate, followed by ester hydrolysis reaction.

Using commercially available methyl bromoacetate (mfd. by Aldrich) is easy and preferred. A compound represented by A'-OH can be obtained by the methods disclosed in JP-A-9-249623 (WO 97/25311) and WO 99/01431. However, in case of 2-hydroxycarbazole, using a commercially available product (mfd. by Aldrich) is easy and preferred. The reaction of a compound represented by A'-OH with methyl bromoacetate is preferably carried out in the presence of a base. The amount of methyl bromoacetate to be used may be generally 1 to 10 equivalents, preferably 1 to 3 equivalents with respect to the compound represented by A'-OH. The base may be potassium carbonate, sodium hydride and the like, with potassium carbonate being preferred. The amount of the base to be used is 1 to 10 equivalents, preferably 2 to 5 equivalents. Examples of the reaction solvent to be used include ether solvents such as diethyl ether, tetrahydrofuran, 1,4-dioxane and the like, ketone solvents such as acetone, methyl ethyl ketone and the like, polar solvents such as N,N-dimethylformamide, N,N-dimethylacetamide and the like, and sulfoxide solvents such as dimethylsulfoxide, sulfolane and the like. A ketone solvent such as acetone, methyl ethyl ketone and the like is preferably used. The reaction is carried out at a temperature of 20 to 100°C, preferably 20 to 60°C. The reaction time is 1 to 24 hours, preferably 1 to 5 hours. After the reaction of a compound represented by A' -OH with methyl bromoacetate is completed, the resulting ester is preferably hydrolyzed by allowing the ester to stand in the presence of a base for a long period of time or accelerating such a hydrolysis reaction under a thermal condition.

When the thus obtained compound of the formula (10) is reacted with a compound of the formula (4), a compound of the formula (4) may be previously converted into its activated form before the reaction with the compound of the formula (10). The amount of the compound of the formula (4) or its activated form to be used may be generally 1 to 10 equivalents, preferably 1 to 3 equivalents with respect to the compound of the formula (10).

The reaction of a compound of the formula (10) with a compound of the formula (4) is generally carried out using a condensing agent. The condensing agent may be N,N'-dicyclohexylcarbodiimide, 1,1'-carbonyldiimidazole, 1-ethyl-3-(3'-dimethylaminopropyl)carbodiimide hydrochloride and the like, with N,N'-dicyclohexylcarbodiimide being preferred.

The condensing agent (N,N'-dicyclohexylcarbodiimide) may be generally used in an amount of 1 to 10 equivalents, preferably 1 to 3 equivalents, with respect to the compound of the formula (4). Examples of the reaction solvent to be used include ether solvents such as diethyl ether, tetrahydrofuran, 1,4-dioxane and the like, ketone solvents such as acetone, methyl ethyl ketone and the like, polar solvents such as N,N-dimethylformamide, N,N-dimethylacetamide and the like, sulfoxide solvents such as dimethylsulfoxide, sulfolane and the like, nitrile solvents such as acetonitrile and the like, ester solvents such as ethyl acetate, n-butyl acetate, methyl acetate, propyl acetate and the like, and halogen-containing solvents such as dichloromethane, chloroform and the like. An ether solvent such as tetrahydrofuran and the like is preferably used. The reaction is carried out at a temperature of 0 to 100°C, preferably 10 to 40°C. The reaction time is 1 to 50 hours, preferably 10 to 30 hours.

When a compound of the formula (10) is reacted with a compound of the formula (4), it is also preferred to previously convert the compound of the formula (10) into its activated form before the reaction with a compound of the formula (4). A method for activating a compound of the formula (10) may be an activated ester method which uses chliro butylcarbonate and the like for converting a compound into its activated form. The other reaction conditions are as set forth above.

The thus obtained reaction product is preferred in that it does not necessarily need a purifying treatment such as column chromatography and a compound of the formula (3) can be obtained after subjecting the reaction product to an easy treatment such as a recrystallizing treatment.

When a compound of the formula (3) is further reduced to obtain a compound of the formula (2), the reducing agent to be used may be borane, lithium aluminium hydride and the like, with borane being preferred. The reducing agent (borane) may be generally used in an amount of 1 to 10 equivalents, preferably 2 to 4 equivalents, with respect to the compound of the formula (3). Examples of the reaction solvent to be used include ether solvents such as diethyl ether, tetrahydrofuran, dioxane and the like, and aromatic solvents such as toluene, xylene and the like. An ether solvent such as tetrahydrofuran and the like is preferably used. The reaction is carried out at a temperature of 0 to 100°C, preferably 10 to 40°C. The reaction time is 1 to 50 hours, preferably 10 to 30 hours

Next, the simultaneous or sequential removals of the protecting groups according to the methods as set forth above can provide a compound of the formula (1).

For example, the deprotecting treatment of a compound of the formula (2) may be carried out using palladium/carbon in the presence of a hydrogen donor. Palladium/carbon may be generally used in an amount of 10 to 50% by weight, preferably 10 to 30% by weight with respect to the compound of the formula (2). Hydrogen gas is used as the hydrogen donor generally at a reaction pressure of atmospheric pressure to 10 atm, preferably atmospheric pressure to 5 atm. Examples of the reaction solvent include alcohol solvents such as methanol, ethanol, i-propanol and the like, and ether solvents such as diethyl ether, tetrahydrofuran, 1,4-dioxane and the like. The preferred reaction solvent is an alcohol solvent such as methanol and the like. The reaction is carried out at a temperature of 0 to 100°C, preferably 10 to 40°C. The reaction time is 1 to 20 hours, preferably 2 to 5 hours.

In each step of the synthesizing routes set forth above, the produced material is preferably purified by a known purifying means, such as column chromatography and the like. However, each intermediate compound is relatively good in crystallinity and can be used in the following reaction step after being subjected to a simple recrystallizing treatment without hard labor. Therefore, the present process, which can save cost and complicated process, is a preferred process. In addition, the present process is also preferred in that each reaction step results in good yield and that the number of reaction steps is relatively few.

### [Preparing Process 2]

The compound of the above-mentioned formula (4) can be reacted with a compound of the formula (15) or its activated form to prepare a compound of the formula (14). The amount of the compound of the formula (15) or its activated form to be added is generally 1 to 10 equivalent, preferably 1 to 3 equivalent, with respect to the compound of the formula (4). The said reaction is preferably carried out in the presence of a base. The base may be an organic base such as triethylamine, diisopropylethylamine and the like, with triethylamine being preferred. The amount of the base to be added is generally I to 10 equivalent, preferably 1 to 5 equivalent. Examples of the reaction solvent to be generally used include ether solvents such as diethyl ether, tetrahydrofuran, 1,4-dioxane and the like, ketone solvents such as acetone, methyl ethyl ketone and the like, polar solvents such as N,N-dimethylformamide, N,N-dimethylacetamide and the like, sulfoxide solvents such as dimethylsulfoxide, sulfolane and the like, nitrile solvents such as acetonitrile and the like, ester solvents such as ethyl acetate, n-butyl acetate, methyl acetate, propyl acetate and the like, and halogen-containing solvents such as dichloromethane, chloroform and the like. An ether solvent such as diethyl ether, tetrahydrofuran, 1,4-dioxane and the like is preferably used. The reaction is generally carried out at a temperature of 0 to 50°C, preferably 0 to 20°C . The reaction time is 1 to 10 hours, preferably 1 to 5 hours.

The leaving group B¹ of a compound of the formula (15) or its activated form may be a halogen atom, in particular, a chlorine or bromine atom. A compound of the formula (15) in the form of acid bromide or acid chloride is preferably selected as the activated form. That is, a compound of the formula (15) or its activated form is preferably chloroacetyl chloride, bromoacetyl chloride or bromoacetyl bromide, which are commercially available (mfd. by Aldrich). Using such commercially available product is easy and preferred.

Next, a compound of the formula (14) can be reacted with a compound represented by A'-OH to prepare a compound of the formula (3). The amount of the compound represented by A'-OH to be used is generally 1 to 10 equivalents, preferably 1 to 3 equivalents, with respect to the compound of the formula (14). The said reaction is preferably carried out in the presence of a base. The base may be sodium hydroxide, potassium hydroxide, potassium carbonate and the like, with potassium carbonate being preferred. Examples of the reaction solvent to be generally used include ether solvents such as diethyl ether, tetrahydrofuran, 1,4-dioxane and the like, ketone solvents such as acetone, methyl ethyl ketone and the like, polar solvents such as N,N-dimethylformamide, N,N-dimethylacetamide and the like, sulfoxide solvents such as dimethylsulfoxide, sulfolane and the like, and ester solvents such as ethyl acetate, n-butyl acetate, methyl acetate, propyl acetate and the like. A ketone solvent such as acetone, methyl ethyl ketone and the like is preferably used. The reaction is carried out at a temperature of 20 to 100°C, preferably 50 to 100°C. The reaction time is 1 to 10 hours, preferably 1 to 5 hours.

The thus obtained compound of the formula (3) can be subjected to the sequential reaction steps according to Preparing Process 1 set forth above to give a compound of the formula (2) and then a compound of the formula (1).

In each step of the synthesizing routes set forth above, the produced material is preferably purified by a known purifying means, such as column chromatography and the like. However, each intermediate compound is relatively good in crystallinity and can be used in the following reaction step after being subjected to a simple recrystallizing treatment without hard labor. Therefore, the present process, which can save cost and complicated process, is a preferred process. In addition, the present process is also preferred in that each reaction step results in good yield and that the number of reaction steps is relatively few.

### [Preparing Process 3]

When a compound of the formula (11) is selected in the process of reacting a compound of the above-mentioned formula (4) with a compound of the formula (11) or (12) set forth above, the reaction product derived from the compound of the formula (11) can be then reduced to provide a compound of the formula (2).

The reaction of a compound of the formula (4) with a compound of the formula (11) is carried out by mixing an acetal compound which is a precursor of a compound of the formula (11) and a compound of the formula (4) in the presence of an acid. The precursor acetal is converted into the corresponding compound of the formula (11). The acid to be used may be hydrochloric acid, p-toluenesulfonic acid, trifluoroacetic acid and the like, with hydrochloric acid being preferred. The amount of the acid to be added is generally 1 to 10 equivalents, preferably 1 to 3 equivalents. Examples of the reaction solvent to be generally used include ether solvents such as diethyl ether, tetrahydrofuran, 1,4-dioxane and the like, ketone solvents such as acetone, methyl ethyl ketone and the like, nitrile solvents such as acetonitrile and the like, ester solvents such as ethyl acetate, n-butyl acetate, methyl acetate, propyl acetate and the like, and halogen-containing solvents such as dichloromethane, chloroform and the like. An ether solvent such as diethyl ether, tetrahydrofuran, 1,4-dioxane and the like is preferably used.

An acetal compound which is a precursor of a compound of the formula (11) can be obtained by reacting a compound represented by A'-OH with commercially available bromoacetaldehyde dimethylacetal or bromoacetaldehyde diethylacetal (mfd. by Aldrich). A compound of the formula (11) is preferably stored in the form of a precursor acetal and converted into the corresponding aldehyde compound prior to or upon the reaction, since a compound of the formula (11) may be easily oxidized.

The reaction conditions for synthesizing such a precursor acetal are as follows. That is, the amount of bromoacetaldehyde dimethylacetal or bromoacetaldehyde diethylacetal to be added is generally 1 to 10 equivalents, preferably 2 to 5 equivalents, with respect to the compound represented by A'-OH. This reaction is preferably carried out in the presence of a base. The base may be sodium hydroxide, potassium hydroxide, potassium carbonate and the like, with potassium carbonate being preferred. The amount of the base to be added is generally 1 to 10 equivalents, preferably 2 to 6 equivalents. Examples of the reaction solvent to be generally used include ether solvents such as diethyl ether, tetrahydrofuran, 1,4-dioxane and the like, ketone solvents such as acetone, methyl ethyl ketone and the like, polar solvents such as N,N-dimethylformamide, N,N-dimethylacetamide and the like, sulfoxide solvents such as dimethylsulfoxide, sulfolane and the like, ester solvents such as ethyl acetate, n-butyl acetate, methyl acetate, propyl acetate and the like, and alcohol solvents such as methanol, ethanol, i-propanol and the like. A polar solvent such as N,N-dimethylformamide and the like is preferably used. The reaction is carried out at a temperature of 20 to 100°C, preferably 30 to 80°C. The reaction time is 5 to 30 hours, preferably 10 to 20 hours.

A compound of the formula (2) can be obtained by reacting a compound of the formula (4) with a compound of the formula (11) and then reducing the reaction product. The reducing agent may be platinum oxide in the presence of a hydrogen donor, sodium borohydride, sodium cyanoborohydride and the like, with platinum oxide in the presence of a hydrogen donor being preferred. The amount of the reducing agent (platinum oxide) to be used may be generally 0.001 to 0.1 equivalent, preferably 0.01 to 0.1 equivalent, with respect to the compound of the formula (4). Hydrogen gas is used as the hydrogen donor generally at a reaction pressure of atmospheric pressure to 10 atm, preferably atmospheric pressure to 5 atm. Examples of the reaction solvent include alcohol solvents such as methanol, ethanol, i-propanol and the like, and ether solvents such as diethyl ether, tetrahydrofuran, 1,4-dioxane and the like. The preferred reaction solvent is an alcohol solvent such as methanol and the like. The reaction is carried out at a temperature of 0 to 100°C, preferably 10 to 40°C. The reaction time is 1 to 50 hours, preferably 10 to 30 hours.

In addition, a compound of the formula (2) can be obtained by reacting a compound of the formula (4) with a compound of the formula (12). This reaction is preferably carried out in the presence of a base. The amount of the compound of the formula (12) to be added may be generally 1 to 5 equivalents, preferably 1 to 2 equivalents, with respect to the compound of the formula (4). The base may be triethylamine, diisopropylethylamine, sodium hydroxide, potassium hydroxide, potassium carbonate and the like, with diisopropylethylamine being preferred. The amount of the base to be added is generally 1 to 10 equivalents, preferably 2 to 5 equivalents. Examples of the reaction solvent to be generally used include ether solvents such as diethyl ether, tetrahydrofuran, 1,4-dioxane and the like, ketone solvents such as acetone, methyl ethyl ketone and the like, polar solvents such as N,N-dimethylformamide, N,N-dimethylacetamide and the like, and ester solvents such as ethyl acetate, n-butyl acetate, methyl acetate, propyl acetate and the like. An ether solvent such as diethyl ether, tetrahydrofuran, 1,4-dioxane and the like is preferably used. The reaction is carried out at a temperature of 0 to 100°C, preferably 20 to 50°C. The reaction time is 1 to 50 hours, preferably 10 to 30 hours.

A compound of the formula (12) can be obtained by reacting a compound represented by A' -OH with commercially available 1,2-bromoethane (mfd. by Aldrich; referred to as a compound the formula (13)). This reaction is preferably carried out in the presence of a base. The amount of the compound of the formula (13) to be used is generally 5 to 50 equivalents, preferably 10 to 50 equivalents, with respect to the compound represented by A'-OH. The base may be sodium hydroxide, potassium hydroxide, potassium carbonate and the like, with potassium carbonate being preferred. The amount of the base to be added is generally 1 to 10 equivalents, preferably 2 to 6 equivalents. Examples of the reaction solvent to be generally used include ether solvents such as diethyl ether, tetrahydrofuran, 1,4-dioxane and the like, ketone solvents such as acetone, methyl ethyl ketone and the like, ester solvents such as ethyl acetate, n-butyl acetate, methyl acetate, propyl acetate and the like, and alcohol solvents such as methanol, ethanol, i-propanol and the like. A polar solvent such as N,N-dimethylformamide and the like is preferably used. The reaction is carried out at a temperature of 20 to 100°C, preferably 30 to 80°C. The reaction time is 5 to 30 hours, preferably 10 to 20 hours.

The thus obtained compound of the formula (2) can be subjected to the reaction steps according to Preparing Process 1 set forth above to give a compound of the formula (1).

In each step of the synthesizing routes set forth above, the produced material is preferably purified by a known purifying means, such as column chromatography and the like. However, each intermediate compound is relatively good in crystallinity and can be used in the following reaction step after being subjected to a simple recrystallizing treatment without hard labor. Therefore, the present process, which can save cost and complicated process, is a preferred process. In addition, the present process is also preferred in that each reaction step results in good yield and that the number of reaction steps is relatively few.

As set forth above, a compound of the formula (1) in which R⁵ is a hydrogen atom can exist in the two different optically active substances. The processes described herein can provide a racemic mixture and also an optically active substance as occasion requires. The reactions set forth above should not alter the relating stereochemistry.

When R⁵ is a hydrogen atom, a mixture of two optical isomers with respect to *1 may be obtained. The mixture can be resolved into the optical isomers as their acid addition salts with an optically active acid such as camphorsulfonic acid, mandelic acid or substituted mandelic acid by a suitable method such as fractional crystallization. Such a fractional crystallization may be carried out using a suitable solvent, preferably a lower alcohol, such as methanol, ethanol, i-propanol or a mixture thereof. Each pair of enantiomers can be resolved into pure isomers by formation of diastereomeric salt, chromatography using an optically active column, or other means. When one of starting materials is optically active, the thus obtained mixture of diastereomers can be resolved into pure isomers by the above-mentioned means.

Salts of a compound of the formula (1) according to the present invention may be a known salt, and examples thereof include hydrochloride, hydrobromide, sulfate, hydrogensulfate, dihydrogen phosphate, citrate, maleate, tartrate, fumarate, gluconate and methanesulfonate, and acid addition salts with an optically active acid such as camphorsulfonic acid, mandelic acid or substituted mandelic acid. Among them, pharmaceutically acceptable salts are particularly preferred. When a compound of the formula (1) is converted into its salt, an acid addition salt of the compound can be obtained by dissolving the compound in an alcohol such as methanol, ethanol and the like, to which the equivalent amount to several times amount of the acid is then added. The acid to be used may be a pharmaceutically acceptable mineral or organic acid, such as hydrochloric acid, hydrobromic acid, sulfuric acid, hydrogensulfate, dihydrogen phosphate, citric acid, maleic acid, tartaric acid, fumaric acid, gluconic acid, methanesulfonic acid and the like.

Further, salts of each compound of the formulae (2), (4), (5) and (9) which is an intermediate of each preparing process may also be preferred.

### Examples

The following examples further illustrate this invention but are not intended to limit it in any way. The high performance liquid chromatography (HPLC) manufactured by Hitachi was used for HPLC measurements. The thin layer chromatography (TLC) used was Precoated silica gel 60 F254 (mfd. by MERCK). The detecting process was carried out with UV (254 nm) irradiation and coloration with ninhydrin. R_{f} values of TLC were obtained on free amines. The column chromatography process was carried out on silica gel 60 (230-400 mesh; mfd. by MERCK). The determination of nuclear magnetic resonance spectrum (NMR) was carried out using Gemini-300 (FT-NMR; mfd. by Varian). The solvent used was CDCl₃ unless otherwise noted. Chemical shift was determined using tetramethylsilane (TMS) as the internal standard and is indicated herein in δ (ppm). Coupling constant is indicated herein in J(Hz). Mass spectrum (MS) was determined by the fast atom bombardment mass spectrometry (FAB-MS) with JEOL-JMS-SX102.

### Example 1

### Synthesis of 2-azido-1-(4-benzyloxy-3-methylsulfonylamino)phenylethanone (Preparation Process 1)

Sodium azide (4.9 g) was added to a solution of2-bromo-1-(4-benzyloxy-3-methylsulfonylamino)phenylethanone (10 g; synthesized by the method reported by A. A. Larsen et al., J. Med. Chem., 10, p. 462 (1967) or C. Kaiser, J. Med. Chem., 7, p. 49 (1974)) in acetone (200 mL). The mixture was then stirred at room temperature for 3.5 hours. After the reaction was completed, the crystal precipitated in the reaction system was filtered off and the solvent was distilled off under reduced pressure. The residue was dissolved in ethyl acetate, and then washed sequentially with a saturated ammonium chloride aqueous solution and water. The organic layer was dried over anhydrous magnesium sulfate and the solvent was distilled off under reduced pressure. The residue was recrystalized from i-propanol (40 mL) and then dried under reduced pressure to give the title compound (8.75 g; yield 96.7%) as a slightly yellowish crystal.
R_{f}: 0.33 (1:2 ethyl acetate/hexane);
¹H-NMR: 2.9-3.0 (3H, s), 4.4-4.6 (2H, s), 5.1-5.3 (2H, s), 6.8-7.0 (1H, bs), 7.0-8.1 (8H, m);
MS: 361 (MH⁺).

### Example 2

### Synthesis of (±)-2-azido-1-(4-benzyloxy-3-methylsulfonylamino)phenylethanol (Preparing Process 1)

Sodium borohydride (472 mg) was added to a suspension of the compound (6 g; obtained in Example 1) in methanol (40 mL). The mixture was then stirred at room temperature for 4.5 hours. After the reaction was completed, the solvent was distilled off under reduced pressure. The residue was dissolved in ethyl acetate and water.
Hydrochloric acid was added until the aqueous layer was neutralized. The organic layer was washed sequentially with water and saturated brine and dried over anhydrous magnesium sulfate. After the solvent was distilled off under reduced pressure, the residue was dried under reduced pressure to give the title compound (6.00 g; yield 99.4%) as a slightly yellowish crystal.
R_{f}: 0.16 (1:9 ethyl acetate/chloroform);
¹H-NMR: 2.9-3.0 (3H, s), 3.4-3.6 (2H, d), 4.8-5.0 (1H, m), 5.0-5.2 (2H, s), 6.7-6.9 (1H, bs), 6.9-7.6 (8H, m);
MS: 363 (MH⁺).

### Example 3

### Synthesis of (±)-2-amino-1-(4-benzyloxy-3-methylsulfonylamino)phenylethanol (Preparing Process 1)

Platinum oxide (85 mg) was added to a solution of the compound (5.0 g; obtained in Example 2) in methanol (30 mL) under an argon atmosphere. The mixture was stirred under a hydrogen atmosphere at atmospheric pressure at room temperature for 22 hours. After the reaction was completed, the atmosphere was replaced with argon and the catalyst was filtered off. The solvent was distilled off under reduced pressure. The residue was recrystalized from diethyl ether and dried under reduced pressure to give the title compound (4.04 g; yield 87%) as a white crystal.
Rf: 0.05 (1:10 methanol/chloroform);
¹H-NMR (DMSO-d₆): 2.5-2.7 (2H, m), 2.8-3.0 (3H, s), 3.2-3.8 (4H, bs), 4.2-4.5 (1H, m), 5.0-5.2 (2H, s), 7.0-7.6 (8H, m);
MS: 337 (MH⁺).

### Example 4

### Synthesis of (±)-2-benzylamino-1-(4-benzyloxy-3-methylsulfonylamino)phenylethanol (Preparing Process 1)

Platinum oxide (107 mg) was added to a solution of the compound (3.15 g; obtained in Example 3) and benzaldehyde (1.0 g) in methanol (45 mL) under an argon atmosphere. The mixture was stirred under a hydrogen atmosphere at atmospheric pressure at room temperature for 15 hours. After the reaction was completed, the atmosphere was replaced with argon and the catalyst was filtered off. The solvent was distilled off under reduced pressure and the residue was purified by silica gel chromatography (1:30 methanol/chloroform) to give the title compound (3.23 g; yield 81%) as an amorphous solid.
R_{f}: 0.29 (1:10 methanol/chloroform);
¹H-NMR (DMSO-d₆): 2.5-2.7 (2H, m), 2.8-3.0 (3H, s), 3.2-3.5 (3H, bs), 3.6-3.8 (2H, s), 4.5-4.7 (1H, m), 5.0-5.2 (2H, s), 7.0-7.6 (13H, m);
MS: 427 (MH⁺).

### Referential Example 1

### Synthesis of (9H-carbazol)-2-yloxyacetic acid

Methyl bromoacetate (5.0 g; mfd. by Aldrich) and potassium carbonate (6.9 g) were added to a solution of 2-hydroxycarbazole (3 g; mfd, by Aldrich) in acetone (30 mL). The mixture was stirred at 50°C for 4 hours. After the reaction was completed, the solvent was distilled off under reduced pressure. The residue was dissolved in methanol (20 mL) and water (20 mL), to which lithium hydroxide (1.2 g) was added. The mixture was stirred at 50 °C for 5 hours and the solvent was distilled off under reduced pressure. The residue was dissolved in ethyl acetate and water. The aqueous layer was acidified with hydrochloric acid, washed with saturated brine and dried over anhydrous magnesium sulfate. The solvent was distilled off under reduced pressure and the residue was recrystalized from ethanol/hexane and dried under reduced pressure to give the title compound (3.08 g; yield 78%) as a slightly yellowish crystal.
R_{f}: 0.05 (1:10 methanol/chloroform);
MS: 242 (MH⁺).

### Example 5

### Synthesis of (±)-N-benzyl-N-[2-hydroxy-2-(4-benzyloxy-3-methylsulfonylamino)phenyl]ethyl (9H-carbazol)-2-yloxyacetamide (Preparing Process 1)

A solution of the compound (283 mg; obtained in Referential Example 1) and N,N'-dicyclohexylcarbodiimide (255 mg; mfd. by Wako Pure Chemical Industries) in tetrahydrofuran (10 mL) was slowly added to a solution of the compound (500 mg; obtained in Example 4) in tetrahydrofuran (10 mL). The mixture was stirred at room temperature for 23 hours. After the reaction was completed, the solvent was distilled off under reduced pressure. The residue was dissolved in ethyl acetate, washed sequentially with water and saturated brine and dried over anhydrous magnesium sulfate. The solvent was then distilled off under reduced pressure. The residue was recrystalized from ethanol/hexane and dried under reduced pressure to give the title compound (468 mg; yield 61.6%) as a white crystal.
R_{f}: 0.29 (1:10 methanol/chloroform);
¹H-NMR (CDCl₃/CD₃OD): 2.7-3.0 (3H, s), 3.2-3.8 (2H, m), 4.2-5.2 (7H, m), 6.6-7.6 (18H, m), 7.6-8.0 (2H, m);
MS: 650 (MH⁺).

### Example 6

### Synthesis of (±)-2-[N-benzyl-N-[2-(9H-carbazol-2-yloxy)]ethyl]amino-1-(4-benzyloxy-3-methylsulfonylamino)phenylethanol (Preparing Process 1)

0.9 M borane/tetrahydrofuran (1.07 mL; mfd. by KANTO CHEMICAL) was added to a solution of the compound (234 mg; obtained in Example 5) in tetrahydrofuran (5 mL) while the solution was cooled with ice-water. The mixture was stirred at room temperature for 17 hours. After the reaction was completed, the solvent was distilled off under reduced pressure. The residue was dissolved in ethyl acetate, washed sequentially with saturated sodium bicarbonate water and saturated brine. The organic layer was dried over anhydrous magnesium sulfate. The solvent was then distilled off under reduced pressure. The residue was purified by silica gel chromatography (1:2 ethyl acetate/hexane) to give the title compound (183 mg; yield 80%) as an amorphous solid.
R_{f}: 0.33 (1:1 ethyl acetate/hexane);
MS: 636 (MH⁺).

### Example 7

### Synthesis of (±)-2-[N-[2-(9H-carbazol-2-yloxy)]ethyl]amino-1-(4-hydroxy-3-methylsulfonylamino)phenylethanol (Preparing Process 1)

10% Palladium/carbon (15 mg) was added to a solution of the compound (150 mg; obtained in Example 6) in methanol (2 mL) under an argon atmosphere. The mixture was stirred under a hydrogen atmosphere at atmospheric pressure at room temperature for 5 hours. After the reaction was completed, the atmosphere was replaced with argon and the catalyst was filtered off. The solvent was distilled off under reduced pressure. The residue was recrystalized from ethanol and dried under reduced pressure to give the title compound (103 mg) as a white crystal.
R_{f}: 0.3 (1:10 methanol/chloroform);
MS: 456 (MH⁺).

The thus obtained compound was shown to be identical to (±)-2-[N-[2-(9H-carbazol-2-yloxy)]ethyl]amino-1-(4-hydroxy-3-methylsulfonylamino)phenylethanol prepared according to the known method (JP-A-9-249623) by the fact that they had the same retention time in HPLC measurements.

### Example 8

### Synthesis of (±)-2-amino-1-(4-benzyloxy-3-methylsulfonylamino)phenylethanol (Preparing Process 1)

Lithium aluminium hydride (632 mg) was added to a solution of the compound (2.0 g; obtained in Example 1) in tetrahydrofuran (50 mL) under an argon atmosphere. The mixture was stirred at room temperature for 20 hours. After the reaction was completed, water (10 mL) and 10% sodium hydroxide (5 mL) were added. The mixture was then filtered, dried over magnesium sulfate and filtered again. The solvent was distilled off under reduced pressure. The residue was recrystalized from diethyl ether and dried under reduced pressure to give the title compound (1.4 g; yield 75%) as a white crystal.
R_{f}: 0.05 (1:10 methanol/chloroform);
MS: 337 (MH⁺).

The thus obtained compound was shown to be identical to (±)-2-amino-1-(4-benzyloxy-3-methylsulfonylamino)phenylethanol obtained in Example 3 by the fact that they had the same retention time in HPLC measurements.

### Example 9

### Synthesis of 2-amino-1-(4-benzyloxy-3-methylsulfonylamino)phenylethanone hydrochloride (Preparation process 1)

Hexamethylenetetramine (2.6 g; mfd. by TOKYO KASEI KOGYO) was added to a solution of 2-bromo-1-(4-benzyloxy-3-methylsulfonylamino)phenylethanone (5 g; synthesized by the method reported by A. A. Larsen et al., J. Med. Chem., 10, p. 462 (1967)) in tetrahydrofuran (100 mL). The mixture was then stirred at room temperature for 15 hours. After the reaction was completed, the crystal precipitated in the reaction system was separated by filtration. Concentrated hydrochloric acid (6.3 mL) was added to a solution of the thus obtained crystal in ethanol (160 mL). The mixture was stirred at 80°C for 5 minutes. After the reaction system was cooled with ice, the crystal precipitated was separated by filtration and dried under reduced pressure to give the title compound (3.4 g; yield 73%) as a white crystal.
R_{f} (as the free amine): 0.1 (1:10 methanol/chloroform);
MS (as the free amine): 335 (MH⁺).

### Example 10

### Synthesis of (±)-2-amino-1-(4-benzyloxy-3-methylsulfonylamino)phenylethanol (Preparing Process 1)

The compound (hydrochloride)(3.4 g; synthesized in Example 9) was added to ethyl acetate (100 mL) and a saturated sodium hydrogen carbonate aqueous solution (100 mL).
The mixture was stirred at room temperature for 30 minutes. The ethyl acetate layer was separated, dried over anhydrous magnesium sulfate and filtered. The solvent was distilled off to give a free amine compound (3 g). Sodium borohydride (255 mg) was added to a suspension of the free amine (3 g) in methanol (20 mL). The mixture was stirred at room temperature for 3 hours. After the reaction was completed, the solvent was distilled off under reduced pressure. The residue was dissolved in ethyl acetate and water.
Hydrochloric acid was added until the aqueous layer was neutralized. The organic layer was washed sequentially with water and saturated brine and dried over anhydrous magnesium sulfate. The solvent was distilled off under reduced pressure and the residue was dried under reduced pressure to give the title compound (2.9 g) as a slightly yellowish crystal.
R_{f}: 0.05 (1:10 methanol/chloroform);
MS: 337 (MH⁺).

The thus obtained compound was shown to be identical to (±)-2-amino-1-(4-benzyloxy-3-methylsulfonylamino)phenylethanol obtained in Example 3 by the fact that they had the same retention time in HPLC measurements.

### Referential Example 2

### Synthesis of [(S,S)-N-methanesulfonyl-1,2-diphenylethylenediamine](p-cymene) ruthenium (S,S)-1,2-diphenylethylenediamine (5.0 g; mfd. by KANKYO KAGAKU CENTER) was dissolved in methylene chloride (25 mL), to which triethylamine (16.4 mL) was added. The mixture was cooled to 0°C under a nitrogen atmosphere. Then, to the mixture was added dropwise methanesulfonyl chloride (1.82 mL; mfd. by Wako Pure Chemical Industries) mixed with methylene chloride (175 mL) over 30 minutes while the internal temperature was maintained at 0 to 5°C. After the adding was completed, the mixture was stirred at 0 to 5°C for 30 minutes.

After the reaction was completed, the solvent was distilled off under reduced pressure.
The white residue was purified by silica gel chromatography (50:1 chloroform/methanol) and then recrystalized from ethanol to give (S,S)-N-methanesulfonyl-1,2-diphenylethylenediamine (3.2 g) as a white powder.
R_{f}: 0.3 (10:1 chloroform/methanol);
¹H-NMR (CDCl₃): 1.51 (2H, br), 2.25 (3H, s), 4.29 (1H, d, J=4.9), 4.55 (1H, d, J=4.9), 5.97 (1H, br), 7.25-7.36 (10H, m).

Next, this compound (3.2 g), dichloro(p-cymene)ruthenium(II) dimer (3.37 g; mfd. by Aldrich) and triethylamine (3.08 mL) were mixed in 2-propanol (100 mL). The mixture was stirred at 80°C for 1 hour. The reaction solution was cooled and the solvent was distilled off under reduced pressure. The residue was washed with water and dried under reduced pressure to give the title compound (6.1 g).

### Example 11

### Synthesis of (R)-2-azido-1-(4-benzyloxy-3-methylsulfonylamino)phenylethanol (Preparing Process 1)

[(S,S)-N-methanesulfonyl-1,2-diphenylethylenediamine](p-cymene) ruthenium (58 mg; synthesized in Referential Example 2 according to the method of Noyori et al., J. Am. Chem. Soc., 117, p. 7562 (1995)) was added to a solution of 2-azido-1-(4-benzyloxy-3-methylsulfonylamino)phenylethanone (3.6 g; synthesized in Example 1) in formic acid/triethylamine (2.5 mL; 5:2 formic acid/triethylamine complex; mfd. by FLUKA) and tetrahydrofuran (6.5 mL). The mixture was stirred at 5°C for 43 hours. After the reaction was completed, the reaction solution was diluted with ethyl acetate, washed sequentially with water and saturated brine and dried over anhydrous magnesium sulfate. The solvent was distilled off under reduced pressure. The residue was then recrystalized from ethanol and dried under reduced pressure to give the title compound (3.44 g; yield 95.0%).
R_{f}: 0.16 (1:9 ethyl acetate/chloroform);
MS: 363 (MH⁺);
HPLC: Optical Purity: 94.2% e.e.

Column: CHIRALCEL OJ (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm.

### Example 12

### Synthesis of (R)-2-amino-1-(4-benzyloxy-3-methylsulfonylamino)phenylethanol (Preparing Process 1)

Under an argon atmosphere, to a solution of chloro(1,5-cyclooctadiene)rhodium(I) dimer (133 mg; mfd. by Wako Pure Chemical Industries) and (2R,4R)-N-(tertbutoxycarbonyl)-4-dicyclohexylphosphino-2-diphenylphosphinomethylpyrrolidine (397 mg; mfd. by FUJI YAKUHIN) in degassed methanol (5 mL) were added 2-amino-1-(4-benzyloxy-3-methylsulfonylamino)phenylethanone hydrochloride (1.0 g; synthesized in Example 9) and triethylamine (2 µL). The mixture was stirred under a hydrogen atmosphere at atmospheric pressure at room temperature for 24 hours. After the reaction was completed, the solvent was distilled off under reduced pressure. The residue was dissolved in ethyl acetate and washed sequentially with saturated sodium bicarbonate water and saturated brine. Organic layer was dried over anhydrous magnesium sulfate and the solvent was distilled off under reduced pressure. The residue was recrystalized from diethyl ether and dried under reduced pressure to give the title compound (854 mg; yield 94.0%) as a white crystal, which was shown to have a high optical purity.
R_{f}: 0.05 (1:10 methanol/chloroform);
MS: 337 (MH⁺).

### Example 13

### Synthesis of (R)-2-[N-[2-(9H-carbazol-2-yloxy)]ethyl]amino-1-(4-hydroxy-3-methylsulfonylamino)phenylethanol (Preparing Process 1)

The title compound was obtained according to Examples 4 to 7. That is, the same reaction as that of Example 4 was carried out, except that (R)-2-amino-1-(4-benzyloxy-3-methylsulfonylamino)phenylethanol (3.15 g; synthesized in Example 12) was used instead of (±)-2-benzylamino-1-(4-benzyloxy-3-methylsulfonylamino)phenylethanol (3.15 g) used in Example 4, to give a reaction product. The resultant product was sequentially treated according to Examples 5, 6 and 7 to give the title compound.
MS: 456 (MH⁺).

The structure of this compound was identified by the retention time in HPLC measurements which was the same retention time as that of the compound obtained in Example 7.

### Example 14

### Synthesis of (±)-2-[N-[2-(9H-carbazol-2-yloxy)]ethyl]amino-1-(3-methylsulfonylamino)phenylethanol (Preparing Process 1)

The title compound was obtained according to Examples 1 to 7. That is, the same reaction as that of Example 1 was carried out, except that 2-bromo-1-(3-methylsulfonylamino)phenylethanone was used instead of 2-bromo-1-(4-benzyloxy-3-methylsulfonylamino)phenylethanone used in Example 1, to give a reaction product. The resultant product was sequentially treated according to Examples 2 to 7 to give the title compound.

The thus obtained compound was shown to be identical to (±)-2-[N-[2-(9H-carbazol-2-yloxy)]ethyl]amino-1-(3-methylsulfonylamino)phenylethanol prepared according to the known method (JP-A-9-249623) by the fact that they had the same retention time in HPLC measurements.

### Example 15

### Synthesis of (±)-2-amino-1-(4-chloro-3-methylsulfonylamino)phenylethanol (Preparing Process 1)

The title compound was obtained according to Examples 1 to 3. That is, the same reaction as that of Example 1 was carried out, except that 2-bromo-1-(4-chloro-3-methylsulfonylamino)phenylethanone (8.2 g; synthesized according to the method described in JP-A-9-249623) was used instead of 2-bromo-1-(4-benzyloxy-3-methylsulfonylamino)phenylethanone (10 g) used in Example 1, to give a reaction product. The resultant product was sequentially treated according to Examples 2 and 3 to give the title compound (3.31 g).
MS: 265 (MH⁺).

### Example 16

### Synthesis of (±)-N-[2-hydroxy-2-(4-chloro-3-methylsulfonylamino)phenyl]ethyl (9H-carbazol)-2-yloxyacetamide (Preparing Process 1)

The title compound was obtained according to Example 5. That is, the same reaction as that of Example 5 was carried out, except that (±)-2-amino-1-(4-chloro-3-methylsulfonylamino)phenylethanol (312 mg; obtained in Example 15) was used instead of ( ±)-2-benzylamino-1-(4-benzyloxy-3-methylsulfonylamino)phenylethanol (500 mg) used in Example 5, to give the title compound (351 mg).
MS: 488 (MH⁺).

### Example 17

### Synthesis of (±)-2-[N-[2-(9H-carbazol-2-yloxy)]ethyl]amino-1-(4-chloro-3-methylsulfonylamino)phenylethanol (Preparing Process 1)

The title compound was obtained according to Example 6. That is, the same reaction as that of Example 6 was carried out, except that (±)-N-[2-hydroxy-2-(4-chloro-3-methylsulfonylamino)phenyl]ethyl (9H-carbazol)-2-yloxyacetamide (176 mg; obtained in Example 16) was used instead of (±)-N-[2-hydroxy-2-(4-benzyloxy-3-methylsulfonylamino)phenyl]ethyl (9H-carbazol)-2-yloxyacetamide (234 mg) used in Example 6, to give the title compound (136 mg).
MS: 474 (MH⁺).

The thus obtained compound was shown to be identical to (±)-2-[N-[2-(9H-carbazol-2-yloxy)]ethyl]amino-1-(4-chloro-3-methylsulfonylamino)phenylethanol prepared according to the known method (JP-A-9-249623) by the fact that they had the same retention time in HPLC measurements.

### Example 18

### Synthesis of (±)-2-amino-1-(4-bromo-3-methylsulfonylamino)phenylethanol (Preparing Process 1)

The title compound was obtained according to Examples 1 to 3. That is, the same reaction as that of Example 1 was carried out, except that 2-bromo-1-(4-bromo-3-methylsulfonylamino)phenylethanone (9.3 g; synthesized according to the method described in JP-A-9-249623) was used instead of 2-bromo-1-(4-benzyloxy-3-methylsulfonylamino)phenylethanone (10 g) used in Example 1, to give a reaction product. The resultant product was sequentially treated according to Examples 2 and 3 to give the title compound (3.76 g).

### Example 19

### Synthesis of (±)-N-[2-hydroxy-2-(4-bromo-3-methylsulfonylamino)phenyl]ethyl (9H-carbazol)-2-yloxyacetamide (Preparing Process 1)

The title compound was obtained according to Example 5. That is, the same reaction as that of Example 5 was carried out, except that (±)-2-amino-1-(4-bromo-3-methylsulfonylamino)phenylethanol (354 mg; obtained in Example 18) was used instead of ( ±)-2-benzylamino-1-(4-benzyloxy-3-methylsulfonylamino)phenylethanol (500 mg) used in Example 5, to give the title compound (399 mg).

### Example 20

### Synthesis of (±)-2-[N-[2-(9H-carbazol-2-yloxy)]ethyl]amino-1-(4-bromo-3-methylsulfonylamino)phenylethanol (Preparing Process 1)

The title compound was obtained according to Example 6. That is, the same reaction as that of Example 6 was carried out, except that (±)-N-[2-hydroxy-2-(4-bromo-3-methylsulfonylamino)phenyl]ethyl (9H-carbazol)-2-yloxyacetamide (200 mg; obtained in Example 19) was used instead of (±)-N-[2-hydroxy-2-(3-methylsulfonylamino-4-benzyloxy)phenyl]ethyl (9H-carbazol)-2-yloxyacetamide (234 mg) used in Example 6, to give the title compound (155 mg).

The thus obtained compound was shown to be identical to (±)-2-[N-[2-(9H-carbazol-2-yloxy)]ethyl]amino-1-(4-bromo-3-methylsulfonylamino)phenylethanol prepared according to the known method (JP-A-9-249623) by the fact that they had the same retention time in HPLC measurements.

### Example 21

### Synthesis of 1-[3-(N-benzyl-N-methylsulfonyl)amino]phenylethanone (preparing Process 2)

Potassium carbonate (7.0 g) was added to a solution of 1-(3-methylsulfonylamino)phenylethanone (10.0 g; synthesized according to the method reported by A. A. Larsen et al., J. Med. Chem., 9, pp. 88-97 (1966)) in acetone (70 mL) with stirring, to which benzyl bromide (8.7 g) and sodium iodide (1.4 g) were then added. The mixture was then warmed up and heated to reflux for 4 hours. The reaction solution was cooled to room temperature, then extracted with ethyl acetate and water, and washed with water. The organic layer was dried over anhydrous magnesium sulfate and the solvent was distilled off under reduced pressure. The thus obtained residue was recrystalized from a mixed solvent (ethyl acetate/hexane) and dried under reduced pressure to give the title compound (11.7 g; yield 82%) as a slightly yellowish crystal.
R_{f}: 0.32 (1:1 ethyl acetate/hexane);
MS: 304 (MH⁺).

### Example 22

### Synthesis of 2-bromo-1-[3-(N-benzyl-N-methylsulfonylamino)]phenylethanone (Preparing Process 2)

A solution of the compound (10.0 g; obtained in Example 21) in methanol (500 mL) was heated to 40°C with stirring. Tetra-n-butylammonium tribromide (17.0 g; mfd. by TOKYO KASEI KOGYO) was added and the mixture was then stirred at 45 to 50°C for 2 hours. The reaction mixture was cooled to 40°C. Hydrobromic acid (140 mL) and water (140 mL) were added and the mixture was stirred at 30 to 40°C for 1 hour. Water (140 mL) was then added to the reaction mixture. This was allowed to cool with stirring for 1 to 2 hours and then cooled with ice. The crystal precipitated was separated by filtration, washed with a mixed solvent of water (300 mL) and methanol (30 mL), and dried under reduced pressure to give the title compound (11.8 g; yield 94%) as a light yellow crystal.
R_{f}: 0.27 (1:1 ethyl acetate/hexane).

### Example 23

### Synthesis of 2-azido-1-[3-(N-benzyl-N-methylsulfonyl)amino]phenylethanone (Preparing Process 2)

Sodium azide (5.1 g) was added to a solution of the compound (10.0 g; obtained in Example 22) in acetone (200 mL). The mixture was stirred at room temperature for 3 hours. After the reaction was completed, the crystal precipitated in the reaction system was filtered off and the solvent was distilled off under reduced pressure. The residue was dissolved in ethyl acetate, and washed sequentially with a saturated ammonium chloride aqueous solution and water. The organic layer was dried over anhydrous magnesium sulfate and the solvent was then distilled off under reduced pressure. The residue was recrystalized from acetone and dried under reduced pressure to give the title compound (8.6 g; yield 96%) as a slightly yellowish crystal.
MS: 345 (MH⁺);
¹H-NMR (DMSO-d₆): 3.14 (3H, s), 4.87 (2H, s), 4.94 (2H, s), 7.19-7.26 (5H, m), 7.28-7.54 (1H, m), 7.62-7.64 (1H, m), 7.78-7.82 (1H, m), 7.93 (1H, d).

### Example 24

### Synthesis of (±)-2-azido-1-[3-(N-benzyl-N-methylsulfonyl)amino]phenylethanol (Preparing Process 2)

Sodium borohydride (247 mg) was added to a suspension of the compound (3 g; obtained in Example 23) in methanol (20 mL) and the mixture was stirred at room temperature for 5 hours. After the reaction was completed, the solvent of the reaction mixture was distilled off under reduced pressure and the residue was then dissolved in ethyl acetate and water. Hydrochloric acid was added until the aqueous layer was neutralized. The organic layer was washed sequentially with water and saturated brine, and dried over anhydrous magnesium sulfate. The solvent was distilled off under reduced pressure and the residue was dried under reduced pressure to give the title compound (2.9 g; yield 97%) as a slightly yellowish crystal.
MS: 347 (MH⁺).

### Example 25

### Synthesis of (±)-2-amino-1-[3-(N-benzyl-N-methylsulfonyl)amino]phenylethanol (Preparing Process 2)

Platinum oxide (53 mg) was added to a solution of the compound (3 g; obtained in Example 24) in methanol under an argon atmosphere. The mixture was then stirred under a hydrogen atmosphere at atmospheric pressure at room temperature for 24 hours. After the atmosphere was replaced with argon, the catalyst was filtered off and the solvent was distilled off under reduced pressure to give the title compound (2.4 g; yield 86%) as a white crystal.
MS: 321 (MH⁺).

### Example 26

### Synthesis of (±)-2-benzylamino-1-[3-(N-benzyl-N-methylsulfonyl)amino]phenylethanol (Preparing Process 2)

Platinum oxide (113 mg) was added to a solution of the compound (3.2 g; obtained in Example 25) and benzaldehyde (1.1 g) in methanol (60 mL) under an argon atmosphere. The mixture was then stirred under a hydrogen atmosphere at atmospheric pressure at room temperature for 18 hours. After the atmosphere was replaced with argon, the catalyst was filtered off and the solvent was distilled off under reduced pressure. The residue was purified by silica gel chromatography to give the title compound (3.5 g; yield 85%).
MS: 411 (MH⁺).

### Example 27

### Synthesis of (±)-N'-benzyl-N'-[2-hydroxy-2-[3-(N-benzyl-N-methylsulfonyl)amino]phenyl)ethyl bromoacetamide (Preparing Process 2)

Triethylamine (1.2 g) was added to a solution of the compound (4.1 g; obtained in Example 26) in tetrahydrofuran (100 mL) with stirring. Bromoacetyl chloride (1.89 g) was added dropwise to the mixture with stirring while the mixture was cooled with ice. The mixture was allowed to warm to room temperature and further stirred for 30 minutes. The reaction mixture was extracted with ethyl acetate and water, and washed with water. The organic layer was dried over anhydrous magnesium sulfate and the solvent was distilled off under reduced pressure. The residue was purified by column chromatography to give the title compound (4.2 g; yield 79%).
MS: 532 (MH⁺).

### Example 28

### Synthesis of (±)-N'-benzyl-N'-[2-hydroxy-2-[3-(N-benzyl-N-methylsulfonyl)amino]phenyl]ethyl (9H-carbazol-2-yloxy)acetamide (Preparing Process 2)

2-Hydroxycarbazole (1.8 g; mfd. by Aldrich) and a 2 N potassium carbonate aqueous solution (10 mL) were added to a solution of the compound (5.3 g; obtained in Example 27) in acetone (60 mL) with stirring. The mixture was stirred at room temperature for 5 hours. The reaction mixture was concentrated under reduced pressure and the residue was extracted with toluene and a sodium hydroxide aqueous solution, and then washed with water. The organic layer was dried over anhydrous magnesium sulfate and the solvent was distilled off under reduced pressure. The residue was purified by column chromatography to give the title compound (4.7 g; yield 74%).
MS: 634 (MH⁺).

### Example 29

### Synthesis of (±)-2-[N'-benzyl-N'-[2-(9H-carbazol-2-yloxy)]ethyl]amino-1-[3-(N-benzyl-N-methylsulfonyl)amino]phenylethanol (Preparing Process 2)

0.9 M borane/tetrahydrofuran (1.85 mL; mfd. by KANTO CHEMICAL) was added to a solution of the compound (634 mg; obtained in Example 28) in tetrahydrofuran (20 mL) while the solution was cooed with ice-water. The mixture was stirred at room temperature for 24 hours. The solvent was distilled off under reduced pressure. The residue was dissolved in ethyl acetate, washed sequentially with saturated sodium bicarbonate water and saturated brine. The organic layer was dried over anhydrous magnesium sulfate. The solvent was then distilled off under reduced pressure. The residue was purified by silica gel chromatography (1:2 ethyl acetate/hexane) to give the title compound (502 mg; yield 81%). R_{f}: 0.5 (2:1 ethyl acetate/hexane);
MS: 620 (MH⁺).

### Example 30

### Synthesis of (±)-2-[N-[2-(9H-carbazol-2-yloxy)]ethyl]amino-1-(3-methylsulfonylamino)phenylethanol (Preparing Process 2)

10% Palladium/carbon (45 mg) was added to a solution of the compound (450 mg; obtained in Example 29) in methanol (10 mL) under an argon atmosphere. The mixture was stirred under a hydrogen atmosphere at atmospheric pressure at room temperature for 5 hours. After the atmosphere was replaced with argon, the catalyst was filtered off and the solvent was distilled off under reduced pressure. The residue was recrystalized from ethanol and dried under reduced pressure to give the title compound (303 mg; yield 95%) as a white crystal.

The thus obtained compound was shown to be identical to (±)-2-[N-[2-(9H-carbazol-2-yloxy)]ethyl]amino-1-(3-methylsulfonylamino)phenylethanol prepared according to the known method (JP-A-9-249623) by the fact that they had the same retention time in HPLC measurements.
R_{f}: 0.32 (1:4 methanol/chloroform);
MS: 440 (MH⁺).

### Example 31

### Synthesis of (±)-2-[N-[2-(9H-carbazol-2-yloxy)]ethyl]amino-1-(3-methylsulfonylamino)phenylethanol (Preparing Process 2)

The title compound was obtained according to Examples 27 to 30. That is, the same reaction as that of Example 27 was carried out, except that (±)-2-amino-1-[3-(N-benzyl-N-methylsulfonyl)amino]phenylethanol (synthesized in Example 25) was used instead of (±)-2-benzylamino-1-[3-(N-benzyl-N-methylsulfonyl)amino]phenylethanol used in Example 27, to give a reaction product. The resultant product was sequentially treated according to Examples 28 to 30 to give the title compound as a white crystal.

The thus obtained compound was shown to be identical to (+)-2-[N-[2-(9H-carbazol-2-yloxy)]ethyl]amino-1-(3-methylsulfonylamino)phenylethanol prepared according to the known method (JP-A-9-249623) by the fact that they had the same retention time in HPLC measurements.
R_{f}: 0.32 (1:4 methanol/chloroform);
MS: 440 (MH⁺).

### Example 32

### Synthesis of (±)-2-[N-[2-(9H-carbazol-2-yloxy)]ethyl]amino-1-(3-methylsulfonylamino)phenylethanol (Preparing Process 2)

The title compound was obtained according to Examples 23 to 30. That is, the same reaction as that of Example 23 was carried out, except that 2-bromo-1-(3-methylsulfonylamino)phenylethanone (synthesized according to the method reported by A. A. Larsen et al., J. Med. Chem., 9, pp. 88-97 (1966)) was used instead of 2-bromo-1-[3-(N-benzyl-N-methylsulfonyl)amino]phenylethanone used in Example 23, to give a reaction product. The resultant product was sequentially treated according to Examples 24 to 30 to give the title compound as a white crystal.

The thus obtained compound was shown to be identical to (±)-2-[N-[2-(9H-carbazol-2-yloxy)]ethyl]amino-1-(3-methylsulfonylamino)phenylethanol prepared according to the known method (JP-A-9-249623) by the fact that they had the same retention time in HPLC measurements.
R_{f}: 0.32 (1:4 methanol/chloroform);
MS: 440 (MH⁺).

### Example 33

### Synthesis of (±)-2-[N-[2-(9H-carbazol-2-yloxy)]ethyl]amino-1-(3-methylsulfonylamino)phenylethanol (Preparing Process 2)

The title compound was obtained according to Examples 23 to 25 and 27 to 29. That is, the same reaction as that of Example 23 was carried out, except that 2-bromo-1-(3-methylsulfonylamino)phenylethanone (synthesized according to the method reported by A. A. Larsen et al., J. Med. Chem., 9, pp. 88-97 (1966)) was used instead of 2-bromo-1-[3-(N-benzyl-N-methylsulfonyl)amino]phenylethanone used in Example 23, to give a reaction product. The resultant product was sequentially treated according to Examples 24, 25 and 27 to 29 to give the title compound.

The thus obtained compound was shown to be identical to (±)-2-[N-[2-(9H-carbazol-2-yloxy)]ethyl]amino-1-(3-methylsulfonylamino)phenylethanol prepared according to the known method (JP-A-9-249623) by the fact that they had the same retention time in HPLC measurements.
R_{f}: 0.32 (1:4 methanol/chloroform);
MS: 440 (MH⁺).

### Example 34

### Synthesis of (±)-2-[N-[2-(9H-carbazol-2-yloxy)]ethyl]amino-1-(4-hydroxy-3-methylsulfonylamino)phenylethanol(Preparing Process 2)

The title compound was obtained according to Examples 27 to 30. That is, the same reaction as that of Example 27 was carried out, except that (±)-2-benzylamino-1-(4-benzyloxy-3-methylsulfonylamino)phenylethanol (synthesized in Example 4) was used instead of (±)-2-benzylamino-1-[3-(N-benzyl-N-methylsulfonyl)amino]phenylethanol used in Example 27, to give a reaction product. The resultant product was sequentially treated according to Examples 28 to 30 to give the title compound as a white crystal.

The thus obtained compound was shown to be identical to (±)-2-[N-[2-(9H-carbazol-2-yloxy)]ethyl]amino-1-(4-hydroxy-3-methylsulfonylamino)phenylethanol prepared according to the known method (JP-A-9-249623) by the fact that they had the same retention time in HPLC measurements.
R_{f}: 0.41 (1:10 methanol/chloroform);
MS: 456 (MH⁺).

### Example 35

### Synthesis of (±)-2-[N-[2-(9H-carbazol-2-yloxy)]ethyl]amino-1-(4-chloro-3-methylsulfonylamino)phenylethanol (Preparing Process 2)

The title compound was obtained according to Examples 27 to 29. That is, the same reaction as that of Example 27 was carried out, except that (±)-2-amino-1-(4-chloro-3-methylsulfonylamino)phenylethanol (synthesized in Example 15) was used instead of (±)-2-benzylamino-1-[3-(N-benzyl-N-methylsulfonyl)amino]phenylethanol used in Example 27, to give a reaction product. The resultant product was sequentially treated according to Examples 28 and 29 to give the title compound.

The thus obtained compound was shown to be identical to (±)-2-[N-[2-(9H-carbazol-2-yloxy)]ethyl]amino-1-(4-chloro-3-methylsulfonylamino)phenylethanol prepared according to the known method (JP-A-9-249623) by the fact that they had the same retention time in HPLC measurements.
MS: 474 (MH⁺).

### Example 36

### Synthesis of (±)-2-[N-[2-(9H-carbazol-2-yloxy)lethyl]amino-1-(4-bromo-3-methylsulfonylamino)phenylethanol (Preparing Process 2)

The title compound was obtained according to Examples 27 to 29. That is, the same reaction as that of Example 27 was carried out, except that (±)-2-amino-1-(4-bromo-3-methylsulfonylamino)phenylethanol (synthesized in Example 18) was used instead of (±)-2-benzylamino-1-[3-(N-benzyl-N-methylsulfonyl)amino]phenylethanol used in Example 27, to give a reaction product. The resultant product was sequentially treated according to Examples 28 and 29 to give the title compound.

The thus obtained compound was shown to be identical to (±)-2-[N-[2-(9H-carbazol-2-yloxy)]ethyl]amino-1-(4-bromo-3-methylsulfonylamino)phenylethanol prepared according to the known method (JP-A-9-249623) by the fact that they had the same retention time in HPLC measurements.
R_{f}: 0.58 (1:3 methanol/ethyl acetate).

### Referential Example 3

### Synthesis of (9H-carbazol-2-yloxy)acetaldehyde (Preparing Process 3)

2-Hydroxycarbazole (2.5 g) and potassium carbonate (9.74 g) were added to N,N-dimethylformamide (50 mL), to which bromoacetaldehyde dimethyl acetal (4.84 mL) and sodium iodide (2.05 g) were then added. The mixture was stirred at room temperature for 1 day, and then extracted with ethyl acetate (50 mL) and water (50 mL). The ethyl acetate layer was dried over anhydrous magnesium sulfate and filtered. Ethyl acetate was distilled off under reduced pressure. Tetrahydrofuran (125 mL) and 3 N hydrochloric acid (13.6 mL) were added to the residue and stirred at room temperature for 16 hours. The mixture was extracted with ethyl acetate (100 mL) and water (50 mL). The ethyl acetate layer was dried over anhydrous magnesium sulfate and filtered. Ethyl acetate was distilled off under reduced pressure. The residue was purified by silica gel column chromatography (1:2 ethyl acetate/hexane) to give the title compound (1.26 g; yield 41%).
MS: 226 (MH⁺).

### Example 37

### Synthesis of (±)-2-[N-[2-(9H-carbazol-2-yloxy)]ethyl]amino-1-(4-benzyloxy-3-methylsulfonylamino)phenylethanol (Preparing Process 3)

The compound (3.364 g; synthesized in Example 3) and the compound (2.252 g; synthesized in Referential Example 3) were added to methanol (50 mL). The mixture was stirred at room temperature for 8 hours. After platinum oxide (110 mg) was added under an argon atmosphere, the mixture was stirred under a hydrogen atmosphere at atmospheric pressure at room temperature for 22 hours. After the atmosphere was replaced with argon, the catalyst was filtered off and the solvent was distilled off under reduced pressure. The residue was recrystalized from diethyl ether and dried under reduced pressure to give the title compound (2.67 g; yield 49%).
MS: 546 (MH⁺).

### Example 38

### Synthesis of (±)-2-[N-[2-(9H-carbazol-2-yloxy)]ethyl]amino-1-(4-hydroxy-3-methylsulfonylamino)phenylethanol (Preparing Process 3)

(± )-2-[N-[2-(9H-carbazol-2-yloxy)]ethyl]amino-1-(4-benzyloxy-3-methylsulfonylamino)phenylethanol synthesized in Example 37 was reacted according to Example 7 to give the title compound.

The thus obtained compound was shown to be identical to the compound obtained in Example 7 by the fact that they had the same R_{f} value in TLC measurements.
R_{f}: 0.3 (1:10 methanol/chloroform).

### Referential Example 4

### Synthesis of (9H-carbazol-2-yloxy)ethyl bromide (Preparing Process 3)

2-Hydroxycarbazole (2.5 g) and potassium carbonate (9.74 g) were added to N,N-dimethylformamide (100 mL), to which 1,2-dibromoethane (38.47 g) was then added. The mixture was stirred at 50°C for 1 day, and then extracted with ethyl acetate (100 mL) and water (100 mL). The ethyl acetate layer was dried over anhydrous magnesium sulfate and filtered. Ethyl acetate was distilled off under reduced pressure. The residue was recrystalized from ethanol (30 mL) and the crystal was filtered to give the title compound (2.73 g; yield 69%).

### Example 39

### Synthesis of (±)-2-[N-benzyl-N-[2-(9H-carbazol-2-yloxy)]ethyl]amino-1-(4-benzyloxy-3-methylsulfonylamino)phenylethanol (Preparing Process 3)

The compound (46.2 mg; synthesized in Example 4), the compound (286 mg; synthesized in Referential Example 4) and N,N-diisopropylethylamine (17.2 µL) were added to tetrahydrofuran (1 mL). The mixture was stirred at room temperature for 8 hours, and then extracted with ethyl acetate (50 mL) and water (50 mL). The ethyl acetate layer was dried over anhydrous magnesium sulfate and filtered. Ethyl acetate was distilled off under reduced pressure. The residue was purified by silica gel column chromatography (1:1 ethyl acetate/hexane) to give the title compound (35.1 mg; yield 51%).
MS: 636 (MH⁺).

### Example 40

### Synthesis of (±)-2-[N-[2-(9H-carbazol-2-yloxy)]ethyl]amino-1-(4-hydroxy-3-methylsulfonylamino)phenylethanol (Preparing Process 3)

(± )-2-[N-benzyl-N-[2-(9H-carbazol-2-yloxy)]ethyl]amino-1-(4-benzyloxy-3-methylsulfonylamino)phenylethanol synthesized in Example 39 was reacted according to Example 7 to give the title compound.

The thus obtained compound was shown to be identical to the compound obtained in Example 7 by the fact that they had the same R_{f} value in TLC measurements.
R_{f}: 0.3 (1:10 methanol/chloroform).

### Example 41

### Synthesis of (±)-2-[N-[2-(9H-carbazol-2-yloxy)]ethyl]amino-1-(3-methylsulfonylamino)phenylethanol (Preparing Process 3)

The title compound was obtained according to Examples 1 to 3 and 37. That is, the same reaction as that of Example 1 was carried out, except that 2-bromo-1-(3-methylsulfonylamino)phenylethanone (synthesized according to the method reported by A. A. Larsen et al., J. Med. Chem., 9, pp. 88-97 (1966)) was used instead of 2-bromo-1-(4-benzyloxy-3-methylsulfonylamino)phenylethanone used in Example 1, to give a reaction product. The resultant product was sequentially treated according to Examples 2 and 3 and then Example 37 to give the title compound.

The thus obtained compound was shown to be identical to (±)-2-[N-[2-(9H-carbazol-2-yloxy)]ethyl]amino-1-(3-methylsulfonylamino)phenylethanol prepared according to the known method (JP-A-9-249623) by the fact that they had the same retention time in HPLC measurements.

### Example 42

### Synthesis of (±)-2-[N-[2-(9H-carbazol-2-yloxy)]ethyl]amino-1-(4-chloro-3-methylsulfonylamino)phenylethanol (Preparing Process 3)

The title compound was obtained according to Example 37. That is, the same reaction as that of Example 37 was carried out, except that (±)-2-amino-1-(4-chloro-3-methylsulfonylamino)phenylethanol (synthesized in Example 15) was used instead of (±)-2-amino-1-(4-benzyloxy-3-methylsulfonylamino)phenylethanol used in Example 37, to give the title compound.

The thus obtained compound was shown to be identical to (±)-2-[N-[2-(9H-carbazol-2-yloxy)]ethyl]amino-1-(4-chloro-3-methylsulfonylamino)phenylethanol prepared according to the known method (JP-A-9-249623) by the fact that they had the same retention time in HPLC measurements.

### Example 43

### Synthesis of (±)-2-[N-[2-(9H-carbazol-2-yloxy)]ethyl]amino-1-(4-bromo-3-methylsulfonylamino)phenylethanol (Preparing Process 3)

The title compound was obtained according to Example 37. That is, the same reaction as that of Example 37 was carried out, except that (±)-2-amino-1-(4-bromo-3-methylsulfonylamino)phenylethanol (synthesized in Example 18) was used instead of (±)-2-amino-1-(4-benzyloxy-3-methylsulfonylamino)phenylethanol used in Example 37, to give the title compound.

The thus obtained compound was shown to be identical to (±)-2-[N-[2-(9H-carbazol-2-yloxy)]ethyl]amino-1-(4-bromo-3-methylsulfonylamino)phenylethanol prepared according to the known method (JP-A-9-249623) by the fact that they had the same retention time in HPLC measurements.

### Example 44

### Synthesis of (R)-2-[N-[2-(9H-carbazol-2-yloxy)]ethyl]amino-1-(4-hydroxy-3-methylsulfonylamino)phenylethanol (Preparing Process 3)

The title compound was obtained according to Examples 37 and 38. That is, the same reaction as that of Example 37 was carried out, except that (R)-2-amino-1-(4-benzyloxy-3-methylsulfonylamino)phenylethanol (synthesized in Example 12) was used instead of (±)-2-amino-1-(4-benzyloxy-3-methylsulfonylamino)phenylethanol used in Example 37, to give a reaction product. The resultant product was further treated according to Example 38 to give the title compound.

The thus obtained compound was shown to be identical to (R)-2-[N-[2-(9H-carbazol-2-yloxy)]ethyl]amino-1-(4-hydroxy-3-methylsulfonylamino)phenylethanol prepared according to the known method (JP-A-9-249623) by the fact that they had the same retention time in HPLC measurements.

### Referential Example 5

### Synthesis of [(S,S)-N-(p-toluenesulfonyl)-1,2-diphenylethylenediamine](p-cymene) ruthenium

Methylene chloride (5 mL) and triethylamine (3.28 mL) were added to (S,S)-1,2-diphenylethylenediamine (1.0 g; mfd. by KANKYO KAGAKU CENTER). To the mixture was added dropwise a solution of p-toluenesulfonyl chloride (916 mg; mfd. by KANTO CHEMICAL) in methylene chloride (10 mL) with stirring under a nitrogen atmosphere at 0°C. The mixture was stirred at 5°C for 17 hours. After the reaction was completed, a 1 N sodium hydroxide aqueous solution (30 mL) was added to the reaction solution. The separated organic layer was dried over anhydrous magnesium sulfate and the solvent was distilled off under reduced pressure. The residue was purified by silica gel chromatography (50:1 chloroform/methanol) to give (S,S)-N-(p-toluenesulfonyl)-1,2-diphenylethylenediamine (1.05 g).

Next, this compound (367 mg), dichloro(p-cymene)ruthenium(II) dimer (306 mg; mfd. by Aldrich) and triethylamine (0.28 mL) were mixed in 2-propanol (7 mL). The mixture was stirred at 80°C for 1 hour. The reaction solution was cooled and the solvent was distilled off under reduced pressure. The residue was washed with water and dried under reduced pressure to give the title compound (637 mg).

### Example 45

### Synthesis of (R)-2-axido-1-(4-benzyloxy-3-methylsulfonylamino)phenylethanol (Preparing Process 1)

[(S,S)-N-(p-toluenesulfonyl)-1,2-diphenylethylenediamine](p-cymene) ruthenium (6.4 mg; synthesized in Referential Example 5) was added to a solution of 2-azido-1-(4-benzyloxy-3-methylsulfonylamino)phenylethanone (180 mg; synthesized in Example 1) in formic acid/triethylamine (0.25 mL; 5:2 formic acid/triethylamine complex; mfd. by FLUKA) and tetrahydrofuran (0.65 mL). The mixture was stirred at 5°C for 2 days. After the reaction was completed, the reaction solution was diluted with ethyl acetate, washed sequentially with water and saturated brine, and dried over anhydrous magnesium sulfate. The solvent was distilled off under reduced pressure. The residue was then recrystalized from ethanol and dried under reduced pressure to give the title compound (170 mg).

The thus obtained compound was shown to be identical to the compound obtained in Example 11 by the fact that they showed the same behavior in TLC and HPLC measurements.
R_{f}:0.16 (1:9 ethyl acetate/chloroform);
HPLC: Optical Purity: 91.4% e.e.

Column: CHIRALCEL OJ (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm.
Column temperature: 25°C.

### Referential Example 6

### Synthesis of [(S,S)-N-(o-toluenesulfonyl)-1,2-diphenylethylenediamine](p-cymene)ruthenium

Methylene chloride (5 mL) and triethylamine (3.28 mL) were added to (S,S)-1,2-diphenylethylenediamine (1.0 g; mfd. by KANKYO KAGAKU CENTER). To the mixture was added dropwise a mixture of o-toluenesulfonyl chloride (670 µL; mfd. by KANTO CHEMICAL) in methylene chloride (35 mL) with stirring under a nitrogen atmosphere at 0°C. The mixture was stirred at 5°C for 17 hours. After the reaction was completed, a 1 N sodium hydroxide aqueous solution (30 mL) was added to the reaction solution. The separated organic layer was dried over anhydrous magnesium sulfate and the solvent was distilled off under reduced pressure. The residue (1.799 g) was purified by silica gel chromatography (50:1 to 20:1 chloroform/methanol) to give (S,S)-N-(o-toluenesulfonyl)-1,2-diphenylethylenediamine (1.05 g).
R_{f}: 0.58 (10:1 chloroform/methanol).

Next, this compound (367 mg), dichloro(p-cymene)ruthenium(II) dimer (306 mg; mfd. by Aldrich) and triethylamine (0.28 mL) were mixed in 2-propanol (7 mL). The mixture was stirred at 80°C for 1 hour. The reaction solution was cooled and the solvent was distilled off under reduced pressure. The residue was washed with water and dried under reduced pressure to give the title compound (615 mg).

### Example 46

### Synthesis of (R)-2-azido-1-(4-benzyloxy-3-methy]sulfonylamino)phenylethano] (Preparing Process 1)

The same reaction as that of Example 45 was carried out for 2 days, except that [(S,S)-N-(o-toluenesulfonyl)-1,2-diphenylethylenediamine](p-cymene) ruthenium (synthesized in Referential Example 6) was used instead of [(S,S)-N-(p-toluenesulfonyl)-1,2-diphenylethylenediamine](p-cymene) ruthenium used in Example 45, to give the title compound.

The thus obtained compound was shown to be identical to the compound obtained in Example 11 by the fact that they showed the same behavior in TLC and HPLC measurements. HPLC: Optical Purity: 91.6% e.e.

Column: CHIRALCEL OJ (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm.
Column temperature: 25°C.

### Referential Example 7

### Synthesis of [(S,S)-N-benzenesulfonyl-1,2-diphenylethylenediamine](p-cymene) ruthenium

Methylene chloride (5 mL) and triethylamine (3.28 mL) were added to (S,S)-1,2-diphenylethylenediamine (1.0 g; mfd. by KANKYO KAGAKU CENTER). To the mixture was added a mixture of benzenesulfonyl chloride (601 µL; mfd. by TOKYO KASEI KOGYO) in methylene chloride (35 mL) with stirring at 0°C. The mixture was stirred at 0°C for 17 hours. After the reaction was completed, the solvent was distilled off under reduced pressure. The residue was purified by silica gel chromatography (50:1 chloroform/methanol) and recrystalized from an ethanol/acetonitrile mixed solvent to give (S,S)-N-benzenesulfonyl-1,2-diphenylethylenediamine (925 mg).
R_{f}: 0.70 (10:1 chloroform/methanol).

Next, this compound (150 mg), dichloro(p-cymene)ruthenium(II) dimer (130 mg; mfd. by Aldrich) and triethylamine (0.11 mL) were mixed in 2-propanol (4 mL). The mixture was stirred at 80°C for 1 hour. The reaction solution was cooled and the solvent was distilled off under reduced pressure. The residue was washed with water and dried under reduced pressure to give the title compound (254 mg).

### Example 47

### Synthesis of (R)-2-azido-1-(4-benzyloxy-3-methylsulfonylamino)phenylethanol (Preparing Process 1)

The same reaction as that of Example 45 was carried out for 2 days, except that [(S,S)-N-benzenesulfonyl-1,2-diphenylethylenediamine](p-cymene) ruthenium (synthesized in Referential Example 7) was used instead of [(S,S)-N-(p-toluenesulfonyl)-1,2-diphenylethylenediamine](p-cymene) ruthenium used in Example 45, to give the title compound.

The thus obtained compound was shown to be identical to the compound obtained in Example 11 by the fact that they showed the same behavior in TLC and HPLC measurements. HPLC: Optical Purity: 91.1% e.e.

Column: CHIRALCEL OJ (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm.
Column temperature: 25°C.

### Referential Example 8

### Synthesis of [(S,S)-N-(4-fluorobenzenesulfonyl)-1,2-diphenylethylenediamine](p-cymene) ruthenium

Methylene chloride (5 mL) and triethylamine (3.28 mL) were added to (S,S)-1,2-diphenylethylenediamine (1.0 g; mfd. by KANKYO KAGAKU CENTER). To the mixture was added a solution of 4-fluorobenzenesulfonyl chloride (0.92 g; mfd. by TOKYO KASEI KOGYO) in methylene chloride (35 mL) with stirring at -3°C. The mixture was stirred at -3 °C for 17 hours. After the reaction was completed, the solvent was distilled off under reduced pressure. The residue was purified by silica gel chromatography (1:1 to 1:2 n-hexane/ethyl acetate) and recrystalized from an ethanol/acetonitrile mixed solvent to give (S,S)-N-(p-fluorobenzenesulfonyl)-1,2-diphenylethylenediamine (1.16 g).
R_{f}: 0.35 (10:1 chloroform/methanol).

Next, this compound (150 mg), dichloro(p-cymene)ruthenium(II) dimer (124 mg; mfd. by Aldrich) and triethylamine (0.113 mL) were mixed in 2-propanol (4 mL). The mixture was stirred at 80°C for 2 hours. The reaction solution was cooled and the solvent was distilled off under reduced pressure. The residue was washed with water and dried under reduced pressure to give the title compound (254 mg).

### Example 48

### Synthesis of (R)-2-azido-1-(4-benzyloxy-3-methylsulfonylamino)phenylethanol (Preparing Process 1)

The same reaction as that of Example 45 was carried out for 3 days, except that [(S,S)-N-(4-fluorobenzenesulfonyl)-1,2-diphenylethylenediamine](p-cymene) ruthenium (synthesized in Referential Example 8) was used instead of [(S,S)-N-(p-toluenesulfonyl)-1,2-diphenylethylenediamine](p-cymene) ruthenium used in Example 45, to give the title compound.

The thus obtained compound was shown to be identical to the compound obtained in Example 11 by the fact that they showed the same behavior in TLC and HPLC measurements. HPLC: Optical Purity: 90.1% e.e.

Column: CHIRALCEL OJ (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm.
Column temperature: 25°C.

### Referential Example 9

### Synthesis of [(S,S)-N-trifluoromethanesulfonyl-1,2-diphenylethylenediamine](p-cymene)ruthenium

Methylene chloride (10 mL) and triethylamine (6.56 mL) were added to (S,S)-1,2-diphenylethylenediamine (2.0 g; mfd. by KANKYO KAGAKU CENTER). To the mixture was added dropwise a mixture of trifluoromethanesulfonyl chloride (1.05 mL; mfd. by TOKYO KASEI KOGYO) in methylene chloride (70 mL) with stirring under a nitrogen atmosphere at 0°C. The mixture was stirred at -10°C for 17 hours. After the reaction was completed, the solvent was distilled off under reduced pressure. The residue was purified by silica gel chromatography (1:1 to 1:5 n-hexane/ethyl acetate) to give (S,S)-N-trifluoromethanesulfonyl-1,2-diphenylethylenediamine (200 mg).
R_{f}: 0.50 (10:1 chloroform/methanol).

Next, this compound (50 mg), dichloro(p-cymene)ruthenium(II) dimer (44.5 mg; mfd. by Aldrich) and triethylamine (40.5 µL) were mixed in 2-propanol (2 mL). The mixture was stirred at 80°C for 1 hour. The reaction solution was cooled and the solvent was distilled off under reduced pressure. The residue was washed with 2-propanol and dried under reduced pressure to give the title compound (64 mg).

### Example 49

### Synthesis of (R)-2-azido-1-(4-benzyloxy-3-methylsulfonylamino)phenylethanol (Preparing Process 1)

The same reaction as that of Example 45 was carried out for 2 days, except that [(S,S)-N-trifluoromethanesulfonyl-1,2-diphenylethylenediamine](p-cymene) ruthenium (synthesized in Referential Example 9) was used instead of [(S,S)-N-(p-toluenesulfonyl)-1,2-diphenylethylenediamine](p-cymene) ruthenium used in Example 45, to give the title compound.

The thus obtained compound was shown to be identical to the compound obtained in Example 11 by the fact that they showed the same behavior in TLC and HPLC measurements. HPLC: Optical Purity: 65.3% e.e.

Column: CHIRALCEL OJ (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm.
Column temperature: 25°C.

### Referential Example 10

### Synthesis of [(S,S)-N-(4-methoxybenzenesulfonyl)-1,2-diphenylethylenediamine](p-cymene) ruthenium

Methylene chloride (10 mL) and triethylamine (3.28 mL) were added to (S,S)-1,2-diphenylethylenediamine (1.0 g; mfd. by KANKYO KAGAKU CENTER). To the mixture was added dropwise a solution of 4-methoxybenzenesulfonyl chloride (0.973 g; mfd. by TOKYO KASEI KOGYO) in methylene chloride (30 mL) with stirring under a nitrogen atmosphere at 0°C. The mixture was stirred at 0°C for 17 hours. After the reaction was completed, the solvent was distilled off under reduced pressure. The residue was purified by silica gel chromatography (50:1 chloroform/methanol) and recrystalized from acetonitrile to give (S,S)-N-(4-methoxybenzenesulfonyl)-1,2-diphenylethylenediamine (0.791 g).
R_{f}: 0.33 (10:1 chloroform/methanol).

Next, this compound (100 mg), dichloro(p-cymene)ruthenium(II) dimer (80 mg; mfd. by Aldrich) and triethylamine (73 µL) were mixed in 2-propanol (3 mL). The mixture was stirred at 80°C for 1 hour. The reaction solution was cooled and the solvent was distilled off under reduced pressure. The residue was washed with water and dried under reduced pressure to give the title compound (141 mg).

### Example 50

### Synthesis of (R)-2-azido-1-(4-benzyloxy-3-methylsulfonylamino)phenylethanol (Preparing Process 1)

The same reaction as that of Example 45 was carried out for 2 days, except that [(S,S)-N-(4-methoxybenzenesulfonyl)-1,2-diphenylethylenediamine](p-cymene) ruthenium (synthesized in Referential Example 10) was used instead of [(S,S)-N-(p-toluenesulfonyl)-1,2-diphenylethylenediamine](p-cymene) ruthenium used in Example 45, to give the title compound.

The thus obtained compound was shown to be identical to the compound obtained in Example 11 by the fact that they showed the same behavior in TLC and HPLC measurements. HPLC: Optical Purity: 92.0% e.e.

Column: CHIRALCEL OJ (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm.
Column temperature: 25°C.

### Referential Example 11

### Synthesis of [(S,S)-N-methanesulfonyl-1,2-diphenylethylenediamine]mesitylene ruthenium

(S,S)-N-methanesulfonyl-1,2-diphenylethylenediamine (100 mg; synthesized according to Referential Example 4), dichloro-mesityleneruthenium(II) dimer (100.6 mg) and triethylamine (96 µL) were mixed in 2-propanol (3 mL). The mixture was stirred at 80°C for 1 hour. The reaction solution was cooled and the solvent was distilled off under reduced pressure. The residue was washed with water and dried under reduced pressure to give the title compound (43 mg).

### Example 51

### Synthesis of (R)-2-azido-1-(4-benzyloxy-3-methylsulfonylamino)phenylethanol (Preparing Process 1)

The same reaction as that of Example 45 was carried out for 2 days, except that [(S,S)-N-methanesulfonyl-1,2-diphenylethylenediamine]mesitylene ruthenium (synthesized in Referential Example 11) was used instead of [(S,S)-N-(p-toluenesulfonyl)-1 ,2-diphenylethylenediamine](p-cymene) ruthenium used in Example 45, to give the title compound.

The thus obtained compound was shown to be identical to the compound obtained in Example 11 by the fact that they showed the same behavior in TLC and HPLC measurements. HPLC: Optical Purity: 91.1% e.e.

Column: CHIRALCEL OJ (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm.
Column temperature: 25°C.

### Referential Example 12

### Synthesis of [(S,S)-N-(p-toluenesulfonyl)-1,2-diphenylethylenediamine]mesitylene ruthenium

(S,S)-N-(p-toluenesulfonyl)-1,2-diphenylethylenediamine (183 mg; synthesized according to Referential Example 5), dichloro-mesityleneruthenium(II) dimer (146 mg) and triethylamine (140 µL) were mixed in 2-propanol (5 mL). The mixture was stirred at 80°C for 1 hour. The reaction solution was cooled and the solvent was distilled off under reduced pressure. The residue was washed with water and dried under reduced pressure to give the title compound (246 mg).

### Referential Example 13

### Synthesis of [(S,S)-N-(2-mesitylenesulfonyl)-1,2-diphenylethylenediamine](p-cymene) ruthenium

Methylene chloride (5 mL) and triethylamine (3.28 mL) were added to (S,S)-1,2-diphenylethylenediamine (1.0 g; mfd. by KANKYO KAGAKU CENTER). To the mixture was added a solution of 2-mesitylenesulfonyl chloride (1.03 g; mfd. by TOKYO KASEI KOGYO) in methylene chloride (35 mL) with stirring at 0°C. The mixture was stirred at 5°C for 17 hours. After the reaction was completed, the reaction solution was washed twice with a 1 N sodium hydroxide aqueous solution (30 mL). The organic layer was dried over anhydrous magnesium sulfate and the solvent was distilled off under reduced pressure. The residue was purified by silica gel chromatography (50:1 chloroform/methanol) and recrystalized from ethanol to give (S,S)-N-(2-mesitylenesulfonyl)-1,2-diphenylethylenediamine (865 mg).
R_{f}: 0.50 (20:1 chloroform/methanol).

Next, this compound (100 mg), dichloro(p-cymene)ruthenium(II) dimer (77.5 mg; mfd. by Aldrich) and triethylamine (70.6 µL) were mixed in 2-propanol (4 mL). The mixture was stirred at 80°C for 1 hour. The reaction solution was cooled and the solvent was distilled off under reduced pressure. The residue was washed with water and dried under reduced pressure to give the title compound (158 mg).

### Example 52

### Synthesis of (R)-2-azido-1-(4-benzyloxy-3-methylsulfonylamino)phenylethanol (Preparing Process 1)

A reaction was carried out for 5 days according to Example 45, except that [(S,S)-N-(p-toluenesulfonyl)-1,2-diphenylethylenediamine]mesitylene ruthenium (synthesized in Referential Example 12) was used instead of [(S,S)-N-(p-toluenesulfonyl)-1,2-diphenylethylenediamine](p-cymene) ruthenium used in Example 45, to give the title compound.

The thus obtained compound was shown to be identical to the compound obtained in Example 11 by the fact that they showed the same behavior in TLC and HPLC measurements. HPLC: Optical Purity: 88.6% e.e.

Column: CHIRALCEL OJ (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm.
Column temperature: 25°C.

### Example 53

### Synthesis of 2-bromo-1-(3-methylsulfonylamino)phenylethanone (Preparing Process 1)

A solution of 1-(3-methylsulfonylamino)phenylethanone (20.0 g; prepared according to the method reported by A. A. Larsen et al., J. Med. Chem., 9, pp. 88-97 (1966)) in methanol (1000 mL) was heated to 40°C with stirring. Tetra-n-butylammonium tribromide (50.9 g; mfd. by TOKYO KASEI KOGYO) was added and the mixture was then stirred at 45 to 50°C for 2 hours. The reaction mixture was cooled to 40°C. Hydrobromic acid (280 mL) and water (280 mL) were added and the mixture was stirred at 30 to 40°C for 1 hour. Water (280 mL) was then added and the reaction mixture was allowed to cool with stirring for 1 to 2 hours and then cooled with ice. The solid precipitated was separated by filtration, and dissolved in ethyl acetate (200 mL). After n-hexane (200 mL) was added, the mixture was stirred at room temperature for 5 hours. The crystal precipitated was filtered, and dried under reduced pressure to give the title compound (12.3 g) as a light yellow crystal.
R_{f}: 0.50 (1:1 ethyl acetate/hexane).

### Example 54

### Synthesis of 2-azido-1-(3-methylsulfonylamino)phenylethanone (Preparing Process 1)

Sodium azide (6.68 g) was added to a solution of the compound (10 g; obtained in Example 53) in acetone (100 mL). The mixture was stirred at room temperature for 2.5 hours. After the reaction was completed, the crystal precipitated in the reaction system was filtered off and the solvent was distilled off under reduced pressure. The residue was dissolved in acetone (45 mL). This was then cooled to precipitate a crystal. Further, n-hexane (150 mL) was added. The crystal was separated by filtration and dried under reduced pressure to give the title compound (7.95 g).
R_{f}: 0.25 (2:1 n-hexane/ethyl acetate; developed twice);
¹H-NMR (DMSO-d₆): 3.15 (3H, s), 4.85 (2H, s), 7.45-7.55 (2H, m), 7.65-7.75 (2H, m), 10.05 (1H, bs);
MS: 255 (MH⁺).

### Example 55

### Synthesis of (R)-2-azido-1-(3-methylsulfonylamino) phenylethanol (Preparing Process 1)

[(S,S)-N-methanesulfonyl-1,2-diphenylethylenediamine](p-cymene) ruthenium (5.8 mg; synthesized in Referential Example 2) was added to a solution of the compound (127 mg; synthesized in Example 54) in formic acid/triethylamine (0.25 mL; 5:2 formic acid/triethylamine complex; mfd. by FLUKA) and tetrahydrofuran (0.65 mL). The mixture was stirred at 5°C for 8 days. After the reaction was completed, the reaction solution was diluted with ethyl acetate, and washed sequentially with water and saturated brine. The organic layer was dried over anhydrous magnesium sulfate and the solvent was distilled off under reduced pressure to give the title compound (115 mg).
R_{f}:0.22 (2:1 n-hexane/ethyl acetate; developed twice);
¹H-NMR (DMSO-d₆): 2.98 (3H, s), 3.30 (2H, bs), 4.75 (1H, bs), 5.85 (1H, bs), 7.05-7.15 (2H, m), 7.24-7.31 (2H, m), 9.72 (1H, bs);
MS: 257 (MH⁺);
HPLC: Optical Purity: 88.7% e.e.

Column: CHIRALCEL OJ (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm.
Column temperature: 25°C.

### Example 56

### Synthesis of (R)-2-[N-[2-(6-hydroxy-9H-carbazol-2-yloxy)]ethyl]amino-1-(3-methylsulfonylamino)phenylethanol hydrochloride (Preparing Process 1)

### Step A. Synthesis of 2-methoxy-6-hydroxycarbazole

Water (30 mL) and concentrated hydrochloric acid (160 mL) were added to 2-nitro-4-methoxyaniline (16.8 g). The mixture was stirred at room temperature for 20 minutes and further at 70°C for 75 minutes. An aqueous solution (30 mL) containing sodium nitrite (11.5 g) was added dropwise to the reaction solution while the reaction solution was maintained at a temperature of 5°C or less with ice cooling. The reaction solution was then stirred for 1 hour while the temperature was maintained at 10°C. The reaction solution was filtered and the residue was washed with water (50 mL). To the filtrate cooled with ice was added dropwise a solution of sodium hydrogencarbonate (123 g) and 1,4-benzoquinone (12.3 g) in water (120 mL) over 1 hour. The reaction solution was then stirred with ice cooling for 4 hours and filtered. The crystal was washed with water and dried. The thus obtained crystal was dissolved in methanol (200 mL) and acetic acid (20 mL) followed by adding 10% palladium/carbon (1.0 g). The mixture was stirred under a hydrogen atmosphere at room temperature for 3 hours. The reaction solution was filtered and the residue was washed with methanol (30 mL). A concentrated aqueous ammonia (50 mL) was added dropwise to the filtrate with ice cooling over 5 minutes. The mixture was then warmed to room temperature and stirred for 12 hours. The reaction solution was filtered and the crystal-was washed with water and then dried in vacuo. The thus obtained crude product was purified by silica gel column chromatography (3:1 to 0:1 hexane/ethyl acetate) to give the title compound (2.71 g).
R_{f}: 0.38 (1:1 ethyl acetate/n-hexane);
¹H-NMR (DMSO-d₆): 3.82 (3H, s), 6.68 (1H, dd, J=2.2, 8.5), 6.77 (1H, dd, J=2.2, 8.5), 6.88 (1H, d, J=2.2), 7.20 (1H, d, J=8.5), 7.30 (1H, d, J=2.2), 7.83 (1H, d, J=8.5), 8.82 (1H, br), 10.73 (1H, br).

### Step B.Synthesis of 2-methoxy-6-benzyloxycarbazole

The compound (3.90 g; synthesized in Step A) was dissolved in acetone (90 mL) and DMF (6 mL), to which potassium carbonate (10.1 g) and benzyl bromide (3.12 g) were added. The mixture was stirred at room temperature for 25 hours. After benzyl bromide (1.56 g) was added, the mixture was further stirred at room temperature for 24 hours. Water (500 mL) was added to the reaction solution and the crystal precipitated was separated by filtration. The residue was washed with water and dried in vacuo. The thus obtained crude product was added to ethyl acetate (40 mL) and the mixture was stirred for 10 minutes. The crystal separated by filtration was dried under reduced pressure to give the title compound (3.28 g).
R_{f}: 0.66 (1:1 ethyl acetate/n-hexane);
¹H-NMR (DMSO-d₆): 3.83 (3H, s), 5.16 (2H, s), 6.73 (1H, dd, J=2.2, 8.5), 6.92 (1H, d, J=2.2), 6.99 (1H, dd, J=2.5, 8.5), 7.30-7.43 (4H, m), 7.50-7.52 (2H, m), 7.67 (1H, d, J=2.2), 7.92 (1H, d, J=8.5), 10.90 (1H, br).

### Step C.Synthesis of 2-hydroxy-6-benzyloxycarbazole

The compound (5.93 g; obtained in Step B) was dissolved in DMSO (110 mL), to which sodium cyanide (5.75 g) was added. The mixture was stirred at 170°C for 7 hours. After water (150 mL) was added, the reaction solution was extracted with ethyl acetate. The organic layer was washed with water and dried. The solvent was distilled off under reduced pressure. The residue was purified by silica gel column chromatography (1:1 hexane/ethyl acetate) to give a 1:1 mixture (1.24 g) of the title compound and 2-methoxy-6-hydroxycarbazole.
R_{f}: 0.69 (1:1 ethyl acetate/n-hexane).
The following is a spectral data of 2-hydroxy-6-benzyloxycarbazole.
¹H-NMR (DMSO-d₆): 5.15 (2H, s), 6.59 (1H, dd, J=2.2, 8.2), 6.76 (1H, d, J=2.5), 6.95 (1H, dd, J=2.5, 8.5), 7.26 (1H, d, J=8.5), 7.32-7.43 (3H, m), 7.49-7.52 (2H, m), 7.60 (1H, d, J=2.5), 7.80 (1H, d, J=8.2), 9.35 (1H, br), 10.72 (1H, br).

### Step D. Synthesis of (R)-2-[N-benzyl-N-[2-(6-benzyloxy-9H-carbazol-2-yloxy)]ethyl]amino-1-[3-(N-benzyl-N-methylsulfonylamino)]phenylethanol

A reaction/treatment in accordance with Referential Example 1 was carried out, except that the 1:1 mixture synthesized in Step C was used instead of 2-hydroxycarbazole to give a mixture of (6-benzyloxy-9H-carbazole)-2-yloxyacetic acid and (2-methoxy-9H-carbazole)-2-yloxyacetic acid.

The said mixture and (R)-2-benzylamino-1-[3-(N-benzyl-N-methylsulfonylamino)]phenylethanol (synthesized from the compound obtained in Example 23 by asymmetric reduction of the compound in accordance with Example 11, then reducing the azido group in accordance with Example 25 and then treating the resulting compound in accordance with Example 26) were subjected to reaction/treatment in accordance with Examples 5 and 6 to give the title compound and (R)-2-[N-benzyl-N-[2-(2-methoxy-9H-carbazol-6-yloxy)]ethyl]amino-1-[3-(N-benzyl-N-methylsulfonylamino)]phenylethanol,

### Step E. Synthesis of (R)-2-[N-[2-(6-hydroxy-9H-carbazol-2-yloxy)]ethyl]amino-1-(3-methylsulfonylamino)phenylethanol hydrochloride

The mixture (2.4 g) of (R)-2-[N-benzyl-N-[2-(6-benzyloxy-9H-carbazol-2-yloxy)]ethyl]amino-1-[3-(N-benzyl-N-methylsulfonylamino)]phenylethanol and the above-mentioned by-product was dissolved in a mixed solvent of tetrahydrofuran (35 mL) and methanol (35 mL), to which acetic acid (2.4 mL) was added. 20% Palladium hydroxide/carbon (1.2 g) was added to the mixture under an argon atmosphere. After the atmosphere was replaced with hydrogen, the mixture was stirred for 15 hours. The catalyst was filtered and washed. The filtrate was placed under reduced pressure to distill off the solvent. The residue was purified by silica gel column chromatography (19:1 to 8:1 chloroform/methanol). An alcoholic 0.5 N hydrochloric acid (3.9 mL) was added to the fraction containing the title compound in the free form and the resultant product was concentrated. The crystal precipitated was separated by filtration, washed with cooled methanol and then dried to give the title compound (370 mg).
¹H-NMR (DMSO-d₆): 3.00 (3H, s), 3.05-3.53 (4H, m), 4.33-4.42 (2H, m), 5.02 (1H, d, J=9.9), 6.27 (1H, br), 6.75 (1H, dd, J=2.2, 8.5), 6.80 (1H, dd, J=2.2, 8.5), 6.95 (1H, d, J=2.2), 7.13-7.24 (3H, m), 7.31-7.39 (3H, m), 7.88 (1H, d, J=8.5), 8.88 (1H, br), 8.99 (1H, br), 9.24 (1H, br), 9.86 (1H, br), 10.85 (1H, br).

## Claims

1. A process for the preparation of a compound of the formula (1): 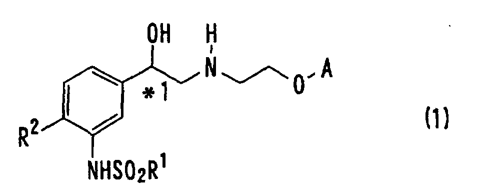 wherein R¹ represents a lower alkyl group or a benzyl group; R² represents a hydrogen atom, a halogen atom or a hydroxyl group; *1 represents an asymmetric carbon atom; and A represents one of the following groups: 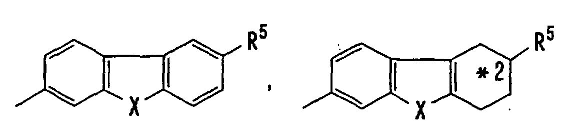 wherein X represents NH, O or S; R⁵ represents a hydrogen atom, a hydroxyl group, an amino group or an acetylamino group; and *2 represents an asymmetric carbon atom when R⁵ is not a hydrogen atom, said process comprising:
(a)
i) azidating a compound of the formula (8): 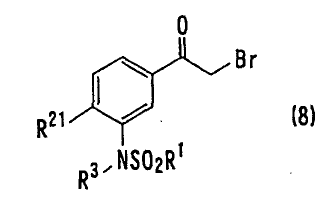 wherein R¹ is as defined above; R²¹ represents a hydrogen atom, a halogen atom, a hydroxyl group or a protected hydroxyl group; R³ represents an amino-protecting group or a hydrogen atom, with an azidating agent to give a compound of the formula (7): 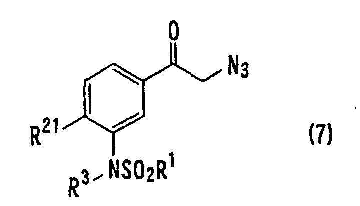 wherein R¹, R²¹ and R³ are as defined above; and then reducing the compound of the formula (7) to give a compound of the formula (5): 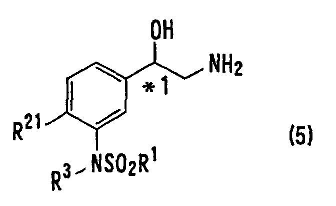 wherein R¹, R²¹, R³ and * 1 are as defined above; or
ii) asymmetrically reducing the compound of the formula (7) to give a compound of the formula (6): 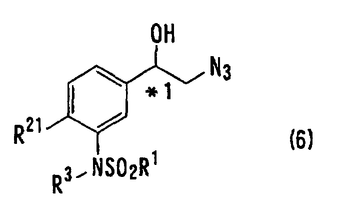 wherein R¹, R²¹, R³ and *1 are as defined above; and then reducing the azido group to give a compound of the formula (5); or alternatively
iii) converting the terminal bromine atom of the side-chain of a compound of the formula (8) into an amino group by amination reaction to give a compound of the formula (9): 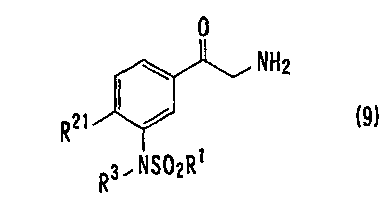 wherein R¹, R²¹ and R³ are as defined above; and then reducing the compound of the formula (9) to give a compound of the formula (5); and then,
(b) where necessary, protecting the compound of the formula (5) obtained in any one of the above steps to give a compound of the formula (4): 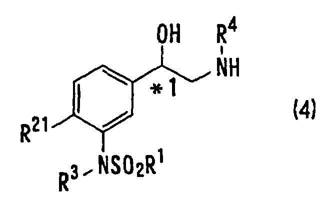 wherein R¹, R²¹, R³ and *1 are as defined above, and R⁴ represents an amino-protecting group or a hydrogen atom; and then,
i) reacting the compound of the formula (4) with a compound of the formula (10): 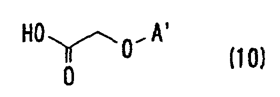 wherein A' represents one of the following groups: 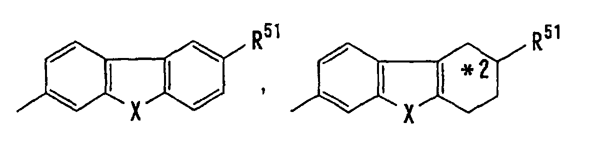 wherein X represents NH, O or S; R⁵¹ represents a hydrogen atom, a protected hydroxyl group, a protected amino group or an acetylamino group; and *2 represents an asymmetric carbon atom when R⁵¹ is not a hydrogen atom, to give a compound of the formula (3): 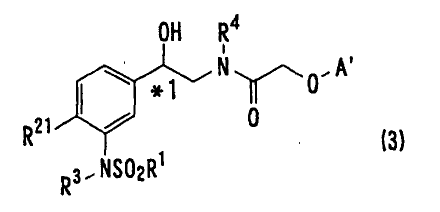 wherein R¹, R²¹, R³, R⁴, A^{'} and * 1 are as defined above; or reacting the compound of the formula (4) with a compound of the formula (15): 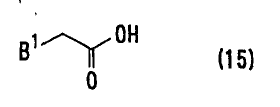 wherein B¹ represents a leaving group, to give a compound of the formula (14): 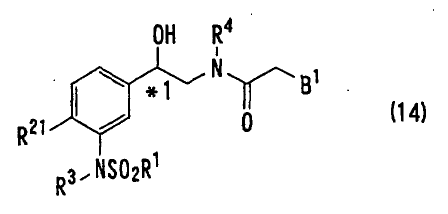 wherein R¹, R²¹, R³, R⁴, B¹ and * 1 are as defined above; and then reacting the resulting compound with a compound represented by A'-OH, wherein A' is as defined above, to give a compound of the formula (3); and then further reducing the compound of the formula (3) obtained in any one of the above steps to give a compound of the formula (2): 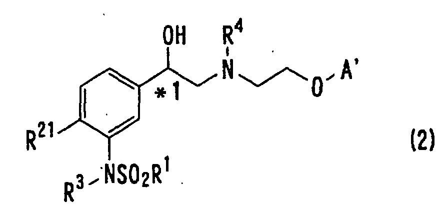 wherein R¹, R²¹, R³, R⁴, A' and *1 are as defined above; or
ii) reacting the compound of the formula (4) with a compound of the formula (11): 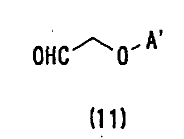 wherein A¹ is as defined above; and then reducing the resulting compound to give a compound of the formula (2); or
iii) reacting the compound of the formula (4) with a compound of the formula (12): 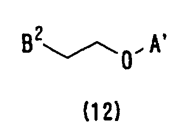
wherein B² represents a leaving group and A' is as defined above, to give a compound of the formula (2); and then,
(c) where necessary, simultaneously or sequentially removing the protecting groups of the compound of the formula (2) obtained in any one of the above steps to give a compound of the formula (1).

2. A process for the preparation of a compound of the formula (1): 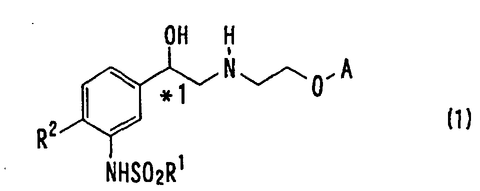 wherein R¹ represents a lower alkyl group or a benzyl group; R² represents a hydrogen atom, a halogen atom or a hydroxyl group; *1 represents an asymmetric carbon atom; and A represents one of the following groups: 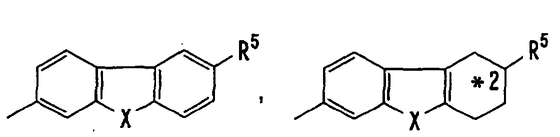 wherein X represents NH, O or S; R⁵ represents a hydrogen atom, a hydroxyl group, an amino group or an acetylamino group; and *2 represents an asymmetric carbon atom when R⁵ is not a hydrogen atom, said process comprising:
(a)
i) azidating a compound of the formula (8): 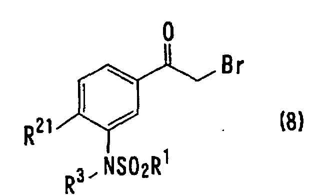 wherein R¹ is as defined above; R²¹ represents a hydrogen atom, a halogen atom, a hydroxyl group or a protected hydroxyl group; R³ represents an amino-protecting group or a hydrogen atom,
with an azidating agent to give a compound of the formula (7): 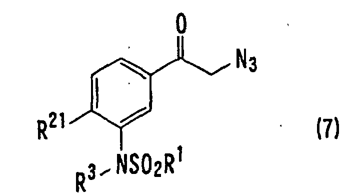 wherein R¹, R²¹ and R³ are as defined above; and then reducing the compound of the formula (7) to give a compound of the formula (5): 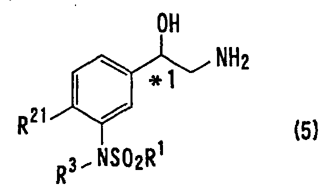 wherein R¹, R²¹, R³ and * 1 are as defined above; or
ii) asymmetrically reducing the compound of the formula (7) to give a compound of the formula (6): 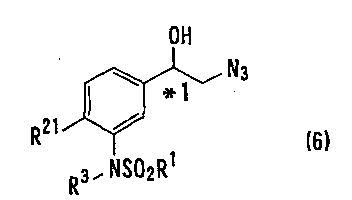 wherein R¹, R²¹, R³ and * 1 are as defined above; and then reducing the azido group to give a compound of the formula (5); or alternatively
iii) converting the terminal bromine atom of the side-chain of a compound of the formula (8) into an amino group by amination reaction to give a compound of the formula (9): 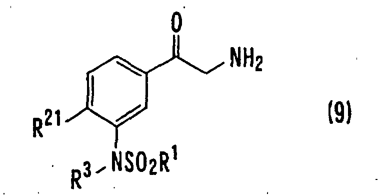 wherein R¹, R²¹ and R³ are as defined above; and then reducing the compound of the formula (9) to give a compound of the formula (5); and then,
(b) where necessary, protecting the compound of the formula (5) obtained in any one of the above steps to give a compound of the formula (4): 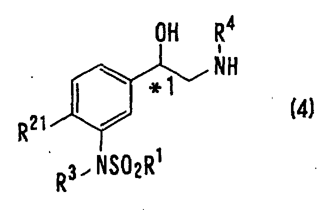 wherein R¹, R²¹, R³ and *1 are as defined above, and R⁴ represents an amino-protecting group or a hydrogen atom; and then,
reacting the compound of the formula (4) with a compound of the formula (10): 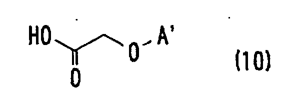 wherein A' represents one of the following groups: 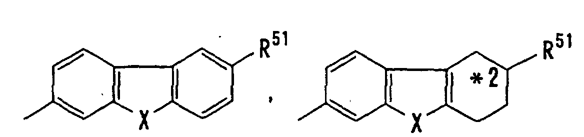 wherein X represents NH, O or S; R⁵¹ represents a hydrogen atom, a protected hydroxyl group, a protected amino group or an acetylamino group; and *2 represents an asymmetric carbon atom when R⁵¹ is not a hydrogen atom, to give a compound of the formula (3): 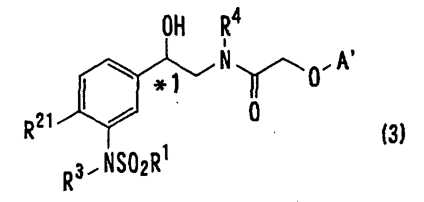 wherein R¹, R²¹, R³, R⁴, A' and *1 are as defined above; and then further reducing the compound of the formula (3) to give a compound of the formula (2): 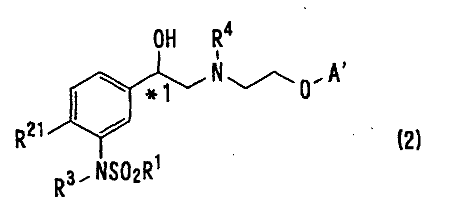 wherein R¹, R²¹, R³, R⁴, A' and * 1 are as defined above; and then,
(c) where necessary, simultaneously or sequentially removing the protecting groups of the compound of the formula (2) to give a compound of the formula (1).

3. A process for the preparation of a compound of the formula (7): 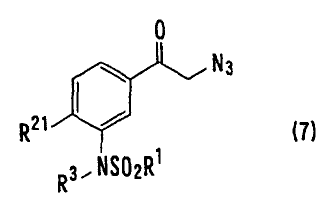 wherein R¹ represents a lower alkyl group or a benzyl group; R²¹ represents a hydrogen atom, a halogen atom, a hydroxyl group or a protected hydroxyl group; and R³ represents an amino-protecting group or a hydrogen atom,
said process comprising:
azidating a compound of the formula (8): 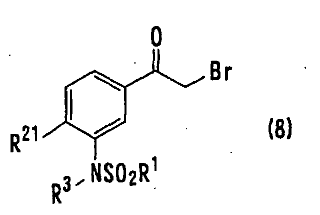 wherein R¹, R²¹ and R³ are as defined above, with an azidating agent to give a compound of the formula (7).

4. A process for the preparation of a compound of the formula (6): 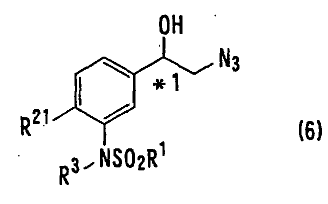 wherein R¹ represents a lower alkyl group or a benzyl group; R²¹ represents a hydrogen atom, a halogen atom, a hydroxyl group or a protected hydroxyl group; R³ represents an amino-protecting group or a hydrogen atom; and * 1 represents an asymmetric carbon atom,
said process comprising:
asymmetrically reducing a compound of the formula (7): 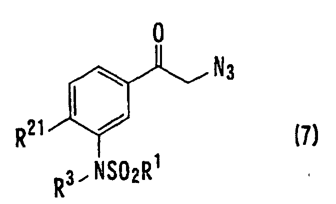 wherein R¹, R²¹ and R³ are as defined above, to give a compound of the formula (6).

5. A compound of the formula (7): 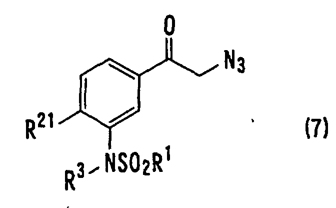 wherein R¹ represents a lower alkyl group or a benzyl group; R²¹ represents a hydrogen atom, a halogen atom, a hydroxyl group or a protected hydroxyl group; and R³ represents an amino-protecting group or a hydrogen atom.

6. A compound of the formula (6): 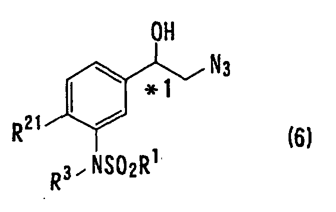 wherein R¹ represents a lower alkyl group or a benzyl group; R²¹ represents a hydrogen atom, a halogen atom, a hydroxyl group or a protected hydroxyl group; R³ represents an amino-protecting group or a hydrogen atom; and * 1 represents an asymmetric carbon atom.

7. A process for the preparation of a compound of the formula (5): 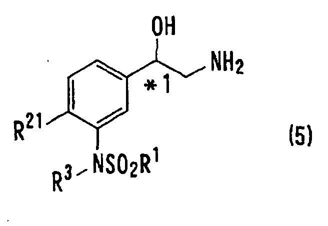 wherein R¹ represents a lower alkyl group or a benzyl group; R²¹ represents a hydrogen atom, a halogen atom, a hydroxyl group or a protected hydroxyl group; R³ represents an amino-protecting group or a hydrogen atom; and *1 represents an asymmetric carbon atom, said process comprising:
reducing a compound of the formula (9): 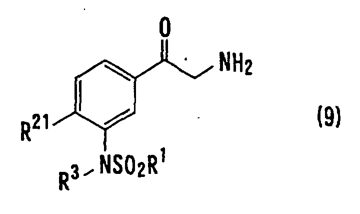 wherein R¹, R²¹ and R³ are as defined above, to give a compound of the formula (5).

8. A compound of the formula (3): 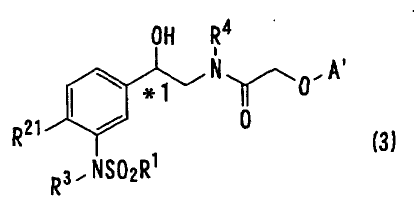 wherein R¹ represents a lower alkyl group or a benzyl group; R²¹ represents a hydrogen atom, a halogen atom, a hydroxyl group or a protected hydroxyl group; R³ represents an amino-protecting group or a hydrogen atom; R⁴ represents an amino-protecting group or a hydrogen atom; * 1 represents an asymmetric carbon atom; and A' represents one of the following groups: 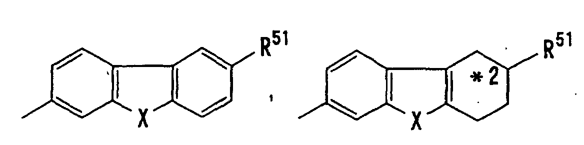 wherein X represents NH, O or S; R⁵¹ represents a hydrogen atom, a protected hydroxyl group, a protected amino group or an acetylamino group; and *2 represents an asymmetric carbon atom when R⁵¹ is not a hydrogen atom.

9. A compound of the formula (14): 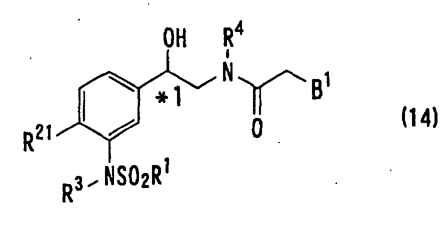 wherein R¹ represents a lower alkyl group or a benzyl group; R²¹ represents a hydrogen atom, a halogen atom, a hydroxyl group or a protected hydroxyl group; R³ represents an amino-protecting group or a hydrogen atom; R⁴ represents an amino-protecting group or a hydrogen atom; B¹ represents a leaving group; and * 1 represents an asymmetric carbon atom.
